(19) Europäisches Patentamt
European Patent Office
Office européen des brevets

(11) **EP 4 748 315 A1**

(12) **EUROPEAN PATENT APPLICATION**

(43) Date of publication:
**27.05.2026 Bulletin 2026/22**

(21) Application number: **24215241.1**

(22) Date of filing: **25.11.2024**

(51) International Patent Classification (IPC):
**A61B 8/00** (2006.01) **A61B 34/20** (2016.01)

(52) Cooperative Patent Classification (CPC):
**A61B 8/4218; A61B 8/4245; A61B 8/54;**
A61B 2034/2055; A61B 2034/2065

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
• **Bruder, Ralf**
  **23562 Lübeck (DE)**
• **Kriebisch, Susann**
  **23562 Lübeck (DE)**

• **Osburg, Jonas**
  **22926 Ahrensburg (DE)**

(72) Inventors:
• **Kriebisch, Susann**
  **23628 Krummesse (DE)**
• **Bruder, Ralf**
  **23562 Lübeck (DE)**
• **Ernst, Floris**
  **23627 Groß Grönau (DE)**

(74) Representative: **Arth, Hans-Lothar**
  **ABK Patent Attorneys**
  **Jasminweg 9**
  **14052 Berlin (DE)**

(54) **AUTONOMOUS ROBOTIC SYSTEM FOR ULTRASOUND IMAGING OF PATIENTS IN ANY POSE**

(57) The present invention relates to an ultrasound imaging system (100) comprising a robotic arm (3), an ultrasound probe (5), at least one depth camera (6) for capturing a depth image of a patient's body (1) and a surrounding, an ultrasound probe pose determination unit (10); a dense pose determination unit (20), and an ultrasound probe pose transformation unit (30), wherein the ultrasound probe position determination unit (10) is configured to determine a six-dimensional pose of the ultrasound probe (5), wherein ultrasound probe pose transformation unit (30) is configured to transform the six-dimensional pose of the ultrasound probe (5) in the depth image to the 3D human body surface model and vice versa using an invertible coordinate transformation function $f(p_{d,probe}) = p_{m,probe}$ obtained by estimating dense correspondences between a selected target region in the depth image of the patient's body and a 3D human body surface model. Said ultrasound imaging system (100) therefore allows recording a position of an ultrasound probe (5) relative to a patient's body (1) autonomous positioning of an ultrasound probe (5) on a patient's body (1) independent of the body position. A further aspect of the present invention is related to a method for recording a six-dimensional pose and/or trajectory of an ultrasound probe (5) relative to a patient's body. A further aspect of the present invention is related to a method for autonomous positioning of an ultrasound probe (5) on a patient's body (1).

Figure 6

## Description

## Field of the invention

The present invention relates to an ultrasound imaging system (100) comprising a robotic arm (3), an ultrasound probe (5), at least one depth camera (6) for capturing a depth image of a patient's body (1) and a surrounding, an ultrasound probe pose determination unit (10); a dense pose determination unit (20), and an ultrasound probe pose transformation unit (30), wherein the ultrasound probe position determination unit (10) is configured to determine a six-dimensional pose of the ultrasound probe (5), wherein ultrasound probe pose transformation unit (30) is configured to transform the six-dimensional pose of the ultrasound probe (5) in the depth image to the 3D human body surface model and vice versa using an invertible coordinate transformation function $f(p_{d,probe}) = p_{m,probe}$ obtained by estimating dense correspondences between a selected target region in the depth image of the patient's body and a 3D human body surface model. Said ultrasound imaging system (100) therefore allows recording a position of an ultrasound probe (5) relative to a patient's body (1) autonomous positioning of an ultrasound probe (5) on a patient's body (1) independent of the body position. A further aspect of the present invention is related to a method for recording a six-dimensional pose and/or trajectory of an ultrasound probe (5) relative to a patient's body. A further aspect of the present invention is related to a method for autonomous positioning of an ultrasound probe (5) on a patient's body (1).

## Background of the invention

Ultrasound (sonography) is a non-ionizing method of examining tissue inside the human body. A probe is lightly pressed against the surface of the skin so that its active surface is directed at the tissue to be examined beneath the surface of the skin. The probe sends a wave into the tissue to be examined and reconstructs a human-interpretable image of the tissue from the reflected echo. In recent years, image quality has greatly improved and, in addition to one- and two-dimensional images, it has become possible to acquire and record volume data at a high sampling rate.

Ultrasound probe placement is critical to image quality. Ultrasound is absorbed and reflected at tissue interfaces. In particular, air pockets between the probe and the surface of the skin or in tissue, such as the lungs or intestines, or bones prevent the ultrasound from penetrating further and thus from imaging deeper layers. But even two good-quality ultrasound images can differ greatly depending on the angle of the probe from which they are taken. To obtain good and at least partially comparable ultrasound images, physicians have to use many different techniques: images are taken from a series of predefined positions and orientations on the human body (ultrasound windows) to ensure an unobstructed view of the target, past the lungs or ribs. The probe is pressed against the skin with variable pressure. The patient is placed in a position that allows the probe to be placed on the target skin surface while taking advantage of gravity and body curvature to optimally deform the body and close air-filled areas between the probe and the target (e.g., lungs in the case of the heart). In the final step, the patient is given commands such as inhalation, exhalation, and muscle tension, which also displace tissues and organs within the body to achieve a better ultrasound image. In addition to optimizing ultrasound image quality, functional imaging (Doppler, M-mode, muscle movements, strain, elastography, etc.) may also require certain sequences of movements that occur during transducer placement and are critical for the desired recording.

Despite the difficulties in positioning the ultrasound probe, ultrasound has a number of advantages over other imaging modalities such as CT or MRI. Ultrasound generates high-resolution volume data in real time, allowing rapid body motion to be captured. Good soft tissue contrast and functional enhancements such as Doppler and elastography provide the basis for a wide range of medical diagnostic procedures. Image-guided interventions are also performed on the basis of ultrasound data. For this reason, fully automated solutions are being researched to simplify the difficult acquisition process. So far, the focus has been on a few individual examinations of lying, possibly immobilized people to simplify transducer placement. The examination has been heavily modified to be performed by a robot.

In particular, the initial placement of an ultrasound probe on the skin surface of any person to be examined is problematic, as is the repeated replacement of the probe over the course of treatment. To do image based ultrasound probe positioning, an ultrasound probe must already be placed on the human body. It must be placed on the desired part of the body with the correct contact pressure and in the desired orientation relative to the body. For ultrasound examinations, a single ultrasound probe position is often not sufficient for the examination. Instead, entire ultrasound probe trajectories on the body surface are required.

The initial placement of the ultrasound probe on the human body with robots is currently solved in the prior art (1) manually, (2) by patient fixation, or (3) automatic patient positioning. For manual placement (1) of the ultrasound probe, an operator positions a robot-mounted ultrasound probe so that it hovers directly over the application area and can be moved to the application area by a defined robot movement, or already touches the skin in the application area. This is done by manual movement of a force-sensitive robot that 'follows the movement' or by teleoperation of the robot via an input device (see Ye, Ruizhong, et al. "Feasibility of a 5G-based robot-assisted remote ultrasound system for cardiopulmonary assessment of patients with coronavirus disease 2019." Chest 159.1 (2021): 270-281). However, the manual intervention of a trained sonographer to place the probe renders the robot superfluous. No costs and time savings can be achieved by manual probe positioning.

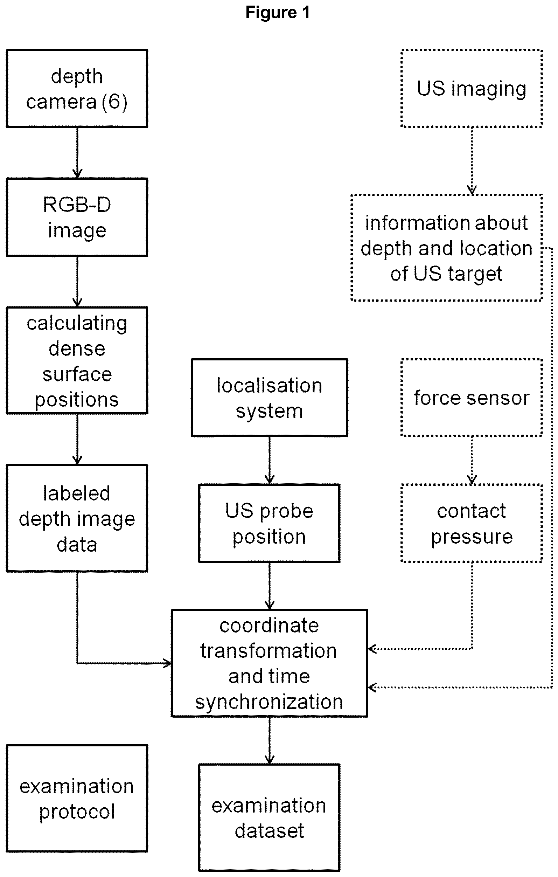

For probe positioning supported by patient fixation (2), the patient is clamped in a holder that spatially limits the application area in such a way that the robot can move to this position as in manual positioning and place the ultrasound probe directly or search for an optimal probe position in a limited area (see for example Figure 1 in Shida, Yuuki, et al. "Automated image acquisition of parasternal long-axis view with robotic echocardiography." IEEE Robotics and Automation Letters (2023)). Patient fixation severely limits the area that can be examined. It may be necessary to fixate the patient in multiple positions for multiple images of different organs, or of one organ from different directions in succession. The robot's workspace is also limited by the fixation device. It must maneuver around the frame. However, this approach still requires the presence of a trained sonographer to guide the patient through the treatment/-diagnostic procedure, thereby cost and time savings are limited.

Automated patient positioning (3) is an approach in radiotherapy, which is transferable to ultrasound examinations. The patient's position is detected optically and a position correction is sent to the patient's bed. A color camera or depth camera captures images of a scene and identifies a person in it by depth values or color values as a contrast to a known background as described in U.S. Patent No. 7,889,906. However, the placement of an ultrasound probe is much more complex than the positioning of an entire patient or body part relative to the radiotherapy system. The probe must be placed very locally, if possible on the area of skin directly underneath. Neither the entire human body nor a single body part needs to be localized precisely, but only this contact area around the probe. The orientation of the probe also plays a crucial role in the ultrasound examination. The probe must always point toward the target being scanned. Contact pressure of the probe plays a critical role in achieving acceptable image quality. Finally, an ultrasound examination does not consist of a single position, but rather follows a trajectory, possibly synchronized with changes in the patient's movement, such as breathing commands. So far, a robotic system that mimics the human ultrasound recording process does not exist is in the prior art.

An important factor for developing a fully automated ultrasound examination system is the tracking and detection of the patient. Formerly, for patient-robot interaction, the patient was immobilized, a preparatory CT volume was generated, or an optical marker was screwed directly into the anatomy to be processed. Nowadays, machine learning methods allow patient tracking at different levels of abstraction: at a low level of detail, body regions, e.g. free skin areas, can be identified and roughly localized (see Szymanski, Mateusz, et al. "Concept Towards Segmenting Arm Areas for Robot-Based Dermatological In Vivo Measurements." 2nd German-West African Conference on Sustainable, Renewable Energy Systems (SusRES2021). TIB Open Publishing, 2021). A more precise localization is provided by so-called skeleton detectors (see Cao, Zhe, et al. "Realtime multi-person 2d pose estimation using part affinity fields." Proceedings of the IEEE conference on computer vision and pattern recognition. 2017), which consider the human body as a tree structure in which body joints are identified as nodes and the location of parts of the human body that can move in relation to each other are allowed as branches of the tree. In addition, artificial 3D models of human bodies (Loper, Matthew, et al. "SMPL: A skinned multi-person linear model." Seminal Graphics Papers: Pushing the Boundaries, Volume 2. 2023. 851-866) can be placed on top of the detected skeletons and parameterized in terms of gender, height, body fat, proportions, etc., so that they fit optimally into the given image material (Bogo, Federica, et al. "Keep it SMPL: Automatic estimation of 3D human pose and shape from a single image." Computer Vision-ECCV 2016: 14th European Conference, Amsterdam, The Netherlands, October 11-14, 2016, Proceedings, Part V 14. Springer International Publishing, 2016.). It is clear that for an optimal fit of the models an almost complete skeleton must be detected and partial occlusions lead to a loss of quality. Modern surface detectors (see Güler, Riza Alp, Natalia Neverova, and Iasonas Kokkinos. "Densepose: Dense human pose estimation in the wild." Proceedings of the IEEE conference on computer vision and pattern recognition. 2018; Wang, Jinbao, et al. "Deep 3D human pose estimation: A review." Computer Vision and Image Understanding 210 (2021): 103225.) assign a position on a virtual patient model to each camera pixel. They work even with partially occluded body parts and with body parts that can be deformed beyond the tree structure, e.g. the thorax with a curved spine.

Surface detectors only assign a body position to pixels of a camera image. Depending on the resolution of the dense body surface pose estimation, in reality it often happens that a predefined probe target point on a body model is not identified in the camera image or is identified multiple times. Also, when recording a trajectory on a human or when continuously positioning a probe with a robot, the point under the probe is always obscured by the probe itself and can never be found. Moreover, a single positively identified position is subject to a high level of noise and systematic error and is unsuitable as a target for a robot. If the orientation of the probe is also considered, it is clear that an orientation can never be determined with respect to a single point. Using surface detectors alone, it is not possible to find a six-dimensional probe pose (position and orientation) on a patient's body, which is necessary for a fully automated ultrasound examination system to place the probe at the right position and in the right orientation on the patient.

Thus, it is the objective of the present invention to provide an autonomous ultrasound imaging system that mimics the human ultrasound recording process and that is able to position and to orientate the ultrasound probe with minimal error on the patient. Particularly, the objective of the present invention is to find a way to identify six-dimensional probe positions relative to a patient's body, even under noise or visual obstruction of the desired position. Moreover, the objective is to provide an autonomous ultrasound imaging system that mimics the human ultrasound acquisition process by positioning and orientating the ultrasound probe along a trajectory with minimal error on the patient. A further objective is the provision of methods for recording ultrasound trajectories on one patient's body and transferring the trajectory to another patient with beforehand unknown different body shape.

The objective of the present invention is solved by the teaching of the independent claims. Further advantageous features, aspects and details of the invention are evident from the dependent claims, the description, the figures, and the examples of the present application.

**Description of the invention**

The present invention is directed to an ultrasound imaging system (100) and methods for recording a patient's position and orientation (pose) and calculating the probe pose relative to the patient. This allows to describe arbitrary probe trajectories locally to the patient and to transfer these trajectories between patients. The transfer enables the probe positioning on the body required for an ultrasound examination to be reproduced on any patient. This represents a major step toward standardization of ultrasound examinations by using the same probe trajectories, thereby leading to time and cost savings for ultrasound examinations, but also should enable comprehensive processing of ultrasound data using artificial intelligence.

Thus, the present invention is directed to an ultrasound imaging system (100) comprising:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body,
- to select a target region on the reference patient body and to identify the densely corresponding target region of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,
- to read a saved six-dimensional pose and/or trajectory of the ultrasound probe (5) on the reference patient body from a computer-readable memory (30), and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body.

The term "six-dimensional pose" or "6D pose" as used herein refers to a six-dimensional vector of the six degrees of freedom of an object in 3D space consisting of 3 position coordinates (or a 3D translation vector) and 3 orientation coordinates (or a 3D rotation vector).

"Depth image" as used herein refers to an image captured with at least one depth camera (6), such as an RGB-D camera or a time-of-flight camera. Thus, a depth image may be an image taken with only one camera or may be an image fused from multiple images captured by multiple depth cameras. As depth cameras usually capture color or grayscale information, a depth image may also comprise a 2D color image (e.g. a 2D-RGB, 2D grayscale or 2D black and white image). Thus, a depth image may be an image only consisting of depth data. A depth image may also be an image comprising of depth data and color data. "Color data" refers to luminance values across multiple channels (e.g. RGB) but also brightness levels in shades of gray, ranging from black to white (grayscale) and black-and-white.

Thus, in one embodiment, the one or more depth image(s) of the patient's body captured with the at least one depth camera (6) may contain only depth data. In another embodiment, the one or more depth image(s) of the patient's body captured with the at least one depth camera (6) may comprise depth data and color data, preferably in the form of a 2D RGB image.

Thus, the present invention is directed to an ultrasound imaging system (100) comprising:

- a robotic arm (3) comprising an end effector (7),
- an ultrasound probe (5) mounted on said end effector (7),
- at least one depth camera (6) for capturing depth images of a patient's body (1) and a surrounding, wherein the depth image comprises depth data and color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body,
- to select a target region on the reference patient body and to identify the densely corresponding target region of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,
- to read a saved six-dimensional pose and/or trajectory of the ultrasound probe (5) on the reference patient body from a computer-readable memory (30), and

- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body.

Thus, the present invention is directed to an ultrasound imaging system (100) comprising:

- a robotic arm (3) comprising an end effector (7),
- an ultrasound probe (5) mounted on said end effector (7),
- at least one depth camera (6) for capturing a depth image of a patient's body (1) and a surrounding, wherein the depth image does not comprise color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body,
- to select a target region on the reference patient body and to identify the densely corresponding target region of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,

- to read a saved six-dimensional pose and/or trajectory of the ultrasound probe (5) on the reference patient body from a computer-readable memory (30), and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body.

Thus, the present invention is directed to an ultrasound imaging system (100) comprising:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and 2D-RGB color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body,
- to select a target region on the reference patient body and to identify the densely corresponding target region of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,

- to read a saved six-dimensional pose and/or trajectory of the ultrasound probe (5) on the reference patient body from a computer-readable memory (30), and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body.

Thus, the present invention is directed to an ultrasound imaging system (100) comprising:

- a robotic arm (3) comprising an end effector (7),
- an ultrasound probe (5) mounted on said end effector (7),
- a depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive a depth image and a luminance-based image of the patient's body captured with the at least one depth camera (6),
- to estimate dense correspondences between the received depth image of the patient's body and a reference patient body,
- to select a target region on the reference patient body and to identify the densely corresponding target region of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by

regression of the selected target region and the densely corresponding target region, wherein $p_d$ is the densely corresponding target region in the depth image and $p_m$ is the selected target region on the reference patient body,

- to read a saved six-dimensional pose and/or trajectory of the ultrasound probe (5) on the reference patient body from a computer-readable memory (30), and

- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body.

The dense pose determination unit (20) of the inventive ultrasound imaging system (100) is configured to estimate dense correspondences between the received depth image of the patient's body and a reference patient body. The dense correspondences estimation or synonymously "dense pose estimation" aims at mapping all human pixels of a depth image to the 3D surface of the human body. In one embodiment, the dense pose determination unit (20) is configured for establishing a relation between a 3D representation of the patient's body (in depth image coordinates) and a 3D representation of a reference patient body (in reference patient body coordinates). In another embodiment, the dense pose determination unit (20) is configured for establishing a relation between a 2D RGB image of the patient's body (in depth image coordinates) and a 3D representation of a reference patient body (in reference patient body coordinates). In another embodiment, the dense pose determination unit (20) is configured for establishing a relation between a 2D RGB image of the patient's body (in depth image coordinates) and a 3D representation of a 3D human body surface model (in model coordinates). In another embodiment, the dense pose determination unit (20) is configured for establishing a relation between a 2D RGB image of the patient's body (in depth image coordinates) and a 3D representation of a 3D human body surface model (in model coordinates) and outputting a 3D human body component index and UV coordinates on the 3D human body surface model so as to map texture information on the RGB image to the 3D human body surface model. In another embodiment, the dense pose determination unit (20) is configured for establishing a relation between a 3D representation of the patient's body (in depth image coordinates) and a 3D representation of a 3D human body surface model (in model coordinates).

Preferably, the 3D representation of the patient's body is a 3D point cloud. Preferably, the 3D representation of the reference patient body is a 3D point cloud. Preferably, the 3D representation of the human body model is a 3D point cloud. In one embodiment, the dense pose determination unit (20) is configured for establishing a relation between a 3D point cloud of the patient's body (in depth image coordinates) and a 3D point cloud of a reference patient body (in reference patient body coordinates). In another embodiment, the dense pose determination unit (20) is configured for establishing a relation between a 2D RGB image of the patient's body (in depth image coordinates) and a 3D point cloud of a reference patient body (in reference patient body coordinates). In another embodiment, the dense pose determination unit (20) is configured for establishing a relation between a 2D RGB image of the patient's body (in depth image coordinates) and a 3D point cloud of a 3D human body surface model (in model coordinates). In another embodiment, the dense pose determination unit (20) is configured for establishing a relation between a 2D RGB image of the patient's body (in depth image coordinates) and a 3D point cloud of a 3D human body surface model (in model coordinates) and outputting a 3D human body component index and UV coordinates on the 3D human body surface model so as to map texture information on the RGB image to the 3D human body surface model. In another embodiment, the dense pose determination unit (20) is configured for establishing a relation between a 3D point cloud of the patient's body (in depth image coordinates) and a 3D point cloud of a 3D human body surface model (in model coordinates).

Thus, the present invention is directed to an ultrasound imaging system (100) comprising:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body,
- to select a target region on the reference patient body and to identify the densely corresponding target region of the patient's body in the received depth image, and
- to generate a 3D point cloud of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,
- to read a saved six-dimensional pose and/or trajectory of the ultrasound probe (5) on the reference patient body from a computer-readable memory (30), and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body.

The term "reference patient body" as used herein, refers to a 3D representation of the body of a reference patient. The reference patient body may be a 3D human body surface model. Also, the reference patient body may be obtained by generating a 3D representation of a reference patient's body from depth image using the inventive ultrasound imaging systems (100) described herein.

The ultrasound probe pose determination unit (10) of the inventive ultrasound imaging system (100) is configured to determine a six-dimensional pose and/or trajectory of the ultrasound probe (5). In one embodiment, the ultrasound probe pose determination unit (10) is configured to determine a six-dimensional pose and trajectory of the ultrasound probe (5). In one embodiment, the ultrasound probe pose determination unit (10) is configured to determine a six-dimensional pose of the ultrasound probe (5), In one embodiment, the ultrasound probe pose determination unit (10) is configured to determine a six-dimensional trajectory of the ultrasound probe (5).

In other words, the ultrasound probe pose determination unit (10) is configured to track the location and orientation of the ultrasound probe (5). Particularly, the ultrasound probe pose determination unit (10) is configured to track a 3-D position and a corresponding 3-D orientation (6D pose) of the ultrasound probe (5). Thus, the ultrasound probe pose determination unit (10) may not only be configured to determine a single six-dimensional pose of the ultrasound probe (5) but may also be configured to determine the six-dimensional pose of the ultrasound probe (5) over time, i.e. the six-dimensional trajectory of the of the ultrasound probe (5). The six-dimensional pose and/or trajectory may be determined from one or more images from the at least on depth camera (6) using 6D pose estimation techniques or by recognition of suitable markers attached to the ultrasound probe (5), or when the ultrasound probe (5) is mounted on an effector of a robotic arm by the joint angle positions of the robotic arm.

The inventive ultrasound imaging system (100) enables the transformation of a 6D ultrasound probe pose from a depth image of a reference patient's body to a depth image of a patient's body. The inventive ultrasound imaging system (100) enables also the transformation of a 6D ultrasound probe pose from a depth image of a patient's body to a 3D human body surface model. The inventive ultrasound imaging system (100) enables further the transformation of a 6D ultrasound probe pose from a 3D human body surface model to a depth image of a patient's body. Said 6D ultrasound probe pose transformation is achieved by determining a transformation function between the selected target region in the depth image of the patient's body and a reference patient body, instead of only mapping a single point between the depth image and reference patient body. The at least one depth camera is used to record the patient and the ultrasound probe position is determined (e.g. by a marker or robot tripod is used to record the probe position and orientation). A selected target region (neighborhood), preferably around the possible covered contact point of the probe, is identified from the point cloud generated by the depth camera. The selected target region is identified in camera coordinates and transferred to the virtual human model by estimating dense correspondences, e.g. with a surface detector.

When recording a trajectory on a human or continuously positioning a probe with a robot, the point under the probe is likely obscured by the probe itself and can never be found. Moreover, a single positively identified position is subject to a high level of noise and systematic error and is unsuitable as a target for a robot. If the orientation of the probe is also taken into account, it is clear that an orientation can never be determined with respect to a single point. Due to the low resolution of body detection, the searched body part may not be found in the image, even if it is present. Using a target region instead of a single point increases the likelihood of identifying the target body part in the image. Second, selecting a body region instead of a body site (or even the entire dense body coverage) allows the transfer of 6D probe positions and orientations (pose) instead of just 3D probe positions. An ultrasound probe must not only rest with its tip on the body, but must also have the desired orientation and contact pressure on the body surface in order to scan ultrasound targets within the body.

By selecting an optimal target region, it is possible to find ultrasound probe poses on humans who are in a completely different posture than before. And it is possible to find the desired probe pose (position and orientation) on a person even when the actual target is hidden from the camera. The latter is usually the case, when the ultrasound probe approaches its target and moves between the camera and the target.

Thus, in one embodiment, the inventive ultrasound imaging system (100) considers a target region (neighborhood) of corresponding surface points around the searched point of contact for the detection and reapproximation of a probe position relative to the human, thereby minimizing noise and mispositionings resulting in a more robust probe positioning and orienting. Numbers of surface points and sizes of the target region directly influence the probe position and orientation estimation in relation to the noise of the corresponding surface point estimation. A reasonable minimum neighborhood size should be chosen to contain at least n points so that random noise of the n positions falls below a desired probe translation accuracy through to averaging. When working on 3d data with surface relationships, the geodetic distance is preferable to Euclidean values for distance calculation, as it only includes contiguous skin surfaces in the neighborhood and not randomly adjacent body parts.

For Gaussian distributed noise, the noise of the averaged point position is calculated, for example, as

$$\sigma_{\mathrm{mean}} = \sigma_{\mathrm{point}}/\sqrt{n}$$

, where $\sigma$ is the standard deviation of the error. The orientational error of the probe is influenced by the noise $\sigma_{\mathrm{mean}}$ and the mean radius $\mathbf{r}_{\mathrm{mean}}$ between points and target region center. For a three-dimensional orientation, a reference must be aligned in at least two spatial axes. $\mathbf{r}_{\mathrm{mean}}$ can therefore be determined as the mean point distance of the second principal axis according to a principal component analysis or approximated as minimum expansion in at least two coordinate axes. Assuming Gaussian noise, the mean rotational error can be approximated for small angles as $\Delta\theta_{\mathrm{mean}} \approx \sigma_{\mathrm{mean}}/\mathbf{r}_{\mathrm{mean}}$, where $\mathbf{r}_{\mathrm{mean}}$ may be chosen depending on a desired radian probe orientation accuracy $\Delta\theta_{\mathrm{mean}}$. Larger neighborhood sizes also minimize systematic errors as they are usually local phenomena.

However, larger neighborhoods also reduce the ability to adapt to body contours and place the ultrasound probe (transducer) precisely on the body surface.

Neighborhoods should therefore only be chosen as large as necessary. For example the selection of a larger neighborhood is beneficial when the direct contact point of the ultrasound probe is covered from the view. The above rules also apply here, but with increasing neighborhood size, the determination of the unseen probe position becomes increasingly unpredictable due to body deformations. The introduction of an upper limit for neighborhood size, above which a probe position is considered non-calculable, is reasonable.

Thus, in one embodiment, the inventive ultrasound imaging system (100) is configured to determine a target region by finding all neighbor points around the probe position below a radius r. Therefore, the present invention is also directed to an ultrasound imaging system (100) comprising:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body,
- to select a target region on the reference patient body by finding all neighbour points around the probe position below a radius *r*, and to identify the densely corresponding target region of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,
- to read a saved six-dimensional pose and/or trajectory of the ultrasound probe (5) on the reference patient body from a computer-readable memory (30), and

- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body.

In another embodiment, the system is configured to determine a target region by finding n nearest neighbor points around the probe position. Thus, the present invention is also directed to an ultrasound imaging system (100) comprising:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body,
- to select a target region on the reference patient body by finding n nearest neighbour points around the probe position, and to identify the densely corresponding target region of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,

- to read a saved six-dimensional pose and/or trajectory of the ultrasound probe (5) on the reference patient body from a computer-readable memory (30), and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body.

In another embodiment, the target region is determined from points neighboring the probe position in an initial area. The area is then expanded incrementally in at least two dimensions by including additional neighbor points, thereby increasing the size of the area with each step until a minimum expansion is met for at least two axis. This approach ensures the target region is both significant in size and balanced across multiple dimensions. Thus, the inventive system is configured to select a target region by finding neighbor points with increasing sizes until a minimum expansion is met in at least two axes. Thus, the present invention is also directed to an ultrasound imaging system (100) comprising:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body,

- to select a target region on the reference patient body by finding neighbour points of the probe position with increasing sizes of the target region until a minimum expansion is met in at least two axes, and to identify the densely corresponding target region of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,
- to read a saved six-dimensional pose and/or trajectory of the ultrasound probe (5) on the reference patient body from a computer-readable memory (30), and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body.

In another embodiment, the target region is determined from points neighboring the probe position in an initial area. The area is then expanded incrementally in at least two dimensions by including additional neighbor points, thereby increasing the size of the area with each step until a mean expansion is met for at least two axis. This approach ensures the target region is both significant in size and balanced across multiple dimensions. Thus, the inventive system is configured to select a target region by finding neighbor points with increasing sizes until a minimum expansion is met in at least two axes. Thus, the present invention is also directed to an ultrasound imaging system (100) comprising:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);

- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body,
- to select a target region on the reference patient body by finding neighbour points of the probe position with increasing sizes of the target region until a mean expansion is met in at least two axes, and to identify the densely corresponding target region of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,
- to read a saved six-dimensional pose and/or trajectory of the ultrasound probe (5) on the reference patient body from a computer-readable memory (30), and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body.

In a further embodiment, the system is configured to identify a selected target region as non-computable by checking if at least one of predefined distance requirements between the probe position and the neighboring points and/or predefined size requirements of the target region is fulfilled.

In a further preferred embodiment, the system is configured to select a target region by a combination of the following methods: finding neighbor points of the probe position with increasing sizes of the target region until a minimum expansion is met in at least two axes, finding neighbor points of the probe position with increasing sizes of the target region until a mean expansion is met in at least two axes, finding n nearest neighbor points around the probe position, and finding all neighbor points around the probe position below a radius *r*.

In a further preferred embodiment, the system is configured to select a target region by a different method when the first method failed to select a computable target region, wherein the different method and the first method are selected from: finding neighbor points of the probe position with increasing sizes of the target region until a minimum expansion is met in at least two axes, finding neighbor points of the probe position with increasing sizes of the target region until a mean expansion is met in at least two axes, finding *n* nearest neighbor points around the probe position, and finding all neighbor points around the probe position below a radius *r*.

The ultrasound imaging system (100) according to the invention is further configured to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body. In contrast to the surface detector of the prior art, the locally invertible function *f* over the selected target region (neighborhood) allows the back and forth transformation of surface model points into camera coordinates of the depth image, which enables the positioning and orientation of the ultrasound probe on different patients of different stature, size, shape, and pose.

The inventive ultrasound imaging system (100) is particularly useful for positioning and orienting an ultrasound probe relative to the patient according to a previously recorded ultrasound probe pose. In other words, the inventive ultrasound imaging system (100) is particularly useful for restoring a recorded ultrasound probe pose relative to a patient. Moreover, the inventive ultrasound imaging system (100) is particularly useful for transferring an ultrasound probe pose from a first patient to a second patient, wherein the first patient and the second patient may differ in stature, size, shape, and pose.

The inventive ultrasound imaging system (100) is particularly useful for executing a previously recorded 6D pose trajectory of an ultrasound probe relative to the patient. Moreover, the inventive ultrasound imaging system (100) is particularly useful for transferring an 6D ultrasound probe pose trajectory from a first patient to a second patient, wherein the first patient and the second patient may differ in stature, size, shape, and pose.

Thus, in a preferred embodiment, the ultrasound imaging system (100) comprises:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional trajectory of the ultrasound probe (5);

wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body,
- to select a target region on the reference patient body and to identify the densely corresponding target region of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,
- to read a saved six-dimensional trajectory of the ultrasound probe (5) on the reference patient body from a computer-readable memory (30), and
- to transform the read six-dimensional trajectory of the ultrasound probe (5) on the reference patient body to the depth image using the function $f^1(p_{m,probe}) = p_{d,probe}$, wherein $f^1$ is the inverted function of *f*,

wherein the robotic arm (3) is configured to execute the transformed six-dimensional trajectory of the ultrasound probe (5) on the patient's body.

In a preferred embodiment, the ultrasound imaging system (100) comprises:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body,
- to select a target region on the reference patient body and to identify the densely corresponding target region of the patient's body in the received depth image, and
- to generate a 3D representation of the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,
- to read a saved six-dimensional pose and/or trajectory of the ultrasound probe (5) on the reference patient body from a computer-readable memory (30), and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body.

In a preferred embodiment, the ultrasound imaging system (100) comprises:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body,
- to select a target region on the reference patient body and to identify the densely corresponding target region of the patient's body in the received depth image, and
- to generate a 3D representation of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,
- to read a saved six-dimensional pose and/or trajectory of the ultrasound probe (5) on the reference patient body from a computer-readable memory (30), and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is

the inverted function of *f*,

wherein the robotic arm (3) is configured to position the ultrasound probe (5) at the transformed six-dimensional pose and/or trajectory on the patient's body.

In a preferred embodiment, the ultrasound imaging system (100) comprises:

- a robotic arm (3) comprising an end effector (7),
- an ultrasound probe (5) mounted on said end effector (7),
- a depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive a depth image of the patient's body captured with the depth camera (6),
- to estimate dense correspondences between the received depth image of the patient's body and a reference patient body,
- to select a target region on the reference patient body and to identify the densely corresponding target region of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,
- to read a saved six-dimensional pose and/or trajectory of the ultrasound probe (5) on the reference patient body from a computer-readable memory (30), and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is

the inverted function of *f*,

wherein the robotic arm (3) is configured to position the ultrasound probe (5) at the transformed six-dimensional pose and/or trajectory on the patient's body.

In a preferred embodiment, the ultrasound imaging system (100) comprises:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a 3D human body surface model,
- to select a target region on the reference patient body and to identify the densely corresponding target region of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the 3D human body surface model,
- to read a saved six-dimensional pose and/or trajectory of the ultrasound probe (5) on the 3D human body surface model from a computer-readable memory (30), and
- to transform the read six-dimensional pose and/or trajectory of the ultrasound probe (5) on the 3D human body surface model to the depth image using the function $f^1(p_{m,probe}) = p_{d,probe}$, wherein $f^1$ is the inverted function of $f$,

wherein the robotic arm (3) is configured to position the ultrasound probe (5) at the transformed six-dimensional pose and/or trajectory on the patient's body.

In a preferred embodiment, the ultrasound imaging system (100) comprises:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a 3D human body surface model,
- to select a target region on the reference patient body and to identify the densely corresponding target region of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the 3D human body surface model,
- to read a saved six-dimensional pose and/or trajectory of the ultrasound probe (5) on the 3D human body surface model from a computer-readable memory (30), and
- to transform the read six-dimensional pose and/or trajectory of the ultrasound probe (5) on the 3D human body surface model to the depth image using the function $f^1(p_{m,probe}) = p_{d,probe}$, wherein $f^1$ is the inverted function of $f$,

wherein the robotic arm (3) is configured to position the ultrasound probe (5) at the transformed six-dimensional pose and/or trajectory on the patient's body and
wherein the 3D human body surface model is a 3D surface model of a generalised human, a 3D surface model of a man, a 3D surface model of a woman, or a 3D surface model of an individual body region.

In a preferred embodiment, the ultrasound imaging system (100) comprises:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body by a surface detector,
- to select a target region on the reference patient body and to identify the densely corresponding target region of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,

- to read a saved six-dimensional pose and/or trajectory of the ultrasound probe (5) on the reference patient body from a computer-readable memory (30), and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body.

In a preferred embodiment, the ultrasound imaging system (100) comprises:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body by a surface detector,
- to select a target region on the reference patient body and to identify the densely corresponding target region of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,
- to read a saved six-dimensional pose and/or trajectory of the ultrasound probe (5) on the reference patient body from a computer-readable memory (30), and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body, and wherein the surface detector comprises a convolutional neural network for feature extraction, a region of interest align region proposal network for generation of region proposals, and a region of interest align layer for refining the region proposals.

In a preferred embodiment, the ultrasound imaging system (100) comprises:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body,
- to select a target region on the reference patient body and to identify the densely corresponding target region of the patient's body in the received depth image, and

- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,
- to read a saved six-dimensional pose and/or trajectory of the ultrasound probe (5) on the reference patient body from a computer-readable memory (30), and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body,

In a preferred embodiment, the ultrasound imaging system (100) comprises:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),
- a display (50) configured to show a sonogram captured with the ultrasound probe (5),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,

- to estimate dense correspondences between the received depth image of the patient's body and a reference patient body,
- to select a target region on the reference patient body and to identify the densely corresponding target region of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,
- to read a saved six-dimensional pose and/or trajectory of the ultrasound probe (5) on the reference patient body from a computer-readable memory (30), and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body.

In a preferred embodiment, the dense pose determination unit (20) is configured to select more than one target region on the 3D human body surface model and to identify the densely corresponding target regions of the patient's body in the one or more received depth image(s) and/or wherein the selected target region comprises a point of contact of the ultrasound probe (5). Thus, another aspect of the present invention is directed to an ultrasound imaging system (100) comprising:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body,
- to select more than one target regions on the reference patient body and to identify more than one densely corresponding target regions of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target regions and the densely corresponding target regions, wherein $p_d$ are the densely corresponding target regions in the depth image and $p_m$ are the selected target regions on the reference patient body,
- to read a saved six-dimensional pose and/or trajectory of the ultrasound probe (5) on the reference patient body from a computer-readable memory (30), and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body, and wherein the selected target region comprises a point of contact of the ultrasound probe (5).

In a preferred embodiment, the ultrasound imaging system (100) comprises:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,

- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body,
- to select a target region on the reference patient body and to identify a densely corresponding target regions of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,
- to read a saved six-dimensional pose and/or trajectory of the ultrasound probe (5) on the reference patient body from a computer-readable memory (30), and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body, and wherein the selected target region comprises a point of contact of the ultrasound probe (5).

In a preferred embodiment, the ultrasound imaging system (100) comprises:

- a robotic arm (3) comprising an end effector (7),
- an ultrasound probe (5) mounted on said end effector (7),

- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body,
- to select more than one target regions on the reference patient body and to identify more than one densely corresponding target regions of the patient's body in the received depth image, and
- to generate a 3D representation of at least the densely corresponding target regions of the patient's body based on the received depth image,

wherein the ultrasound probe pose transformation unit (30) is configured

- to determine an invertible six-dimensional coordinate transformation function $f(p_d) = p_m$ by regression of the selected target regions and the densely corresponding target regions, wherein $p_d$ are the densely corresponding target regions in the depth image and $p_m$ are the selected target regions on the reference patient body,
- to read a saved six-dimensional pose and/or trajectory of the ultrasound probe (5) on the reference patient body from a computer-readable memory (30), and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body.

In a preferred embodiment, the ultrasound imaging system (100) comprises:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body,
- to select a target region on the reference patient body and to identify a densely corresponding target regions of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,
- to read a saved six-dimensional pose and/or trajectory of the ultrasound probe (5) on the reference patient body from a computer-readable memory (30), and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body, and wherein the coordinate transformation function $f$ is a non-linear transformation or a linear transformation

$$f(p_d) = A \cdot p_d + t$$

wherein $A \in \mathbb{R}^{3 \times 3}$ represents a transformation matrix and $t \in \mathbb{R}^3$ represents a translation part.

In preferred embodiments, $A$ may be a product of rotation matrices $A = R_x \cdot R_y \cdot R_z$ or a similarity transformation with $A = S_x \cdot S_y \cdot S_z$.

In a further embodiment, the coordinate transformation function $f$ is a rigid transformation or an affinity transformation. In a further embodiment, the coordinate transformation function $f$ is a non-linear transformation, preferably a B-spline function.

In another embodiment, the dense pose determination unit (20) is configured to select a target region within a geodetic distance of about 1 cm to about 30 cm, more preferably of about 1.5 cm to about 27 cm, more preferably of about 2 cm to about 25 cm, more preferably of about 2.5 cm to about 20 cm, more preferably of about 2.7 cm to about 15 cm, more preferably of about 2.8 cm to about 12 cm, more preferably of about 2.9 cm to about 11 cm, and most preferably of about 3 cm to about 10 cm.

In a preferred embodiment, the ultrasound imaging system (100) comprises:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body,
- to select a target region on the reference patient body within a geodetic distance of about 1 cm to about 30 cm, more preferably of about 1.5 cm to about 27 cm, more preferably of about 2 cm to about 25 cm, more preferably of about 2.5 cm to about 20 cm, more preferably of about 2.7 cm to about 15 cm, more preferably of about 2.8 cm to about 12 cm, more preferably of about 2.9 cm to about 11 cm, and most preferably of about 3 cm to about 10 cm and to identify a densely corresponding target regions of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,
- to read a saved six-dimensional pose and/or trajectory of the ultrasound probe (5) on the reference patient body from a computer-readable memory (30), and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body.

In a preferred embodiment, the ultrasound imaging system (100) comprises:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),

- optionally to fuse the one or more depth images to a single depth image,
- to estimate dense correspondences between the received depth image of the patient's body and a reference patient body,
- to select a target region on the reference patient body within a geodetic distance of about 1 cm to about 30 cm, more preferably of about 1.5 cm to about 27 cm, more preferably of about 2 cm to about 25 cm, more preferably of about 2.5 cm to about 20 cm, more preferably of about 2.7 cm to about 15 cm, more preferably of about 2.8 cm to about 12 cm, more preferably of about 2.9 cm to about 11 cm, and most preferably of about 3 cm to about 10 cm of the six-dimensional pose of the ultrasound probe (5) and to identify a densely corresponding target regions of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,
- to read a saved six-dimensional pose and/or trajectory of the ultrasound probe (5) on the reference patient body from a computer-readable memory (30), and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body.

In a preferred embodiment, the ultrasound imaging system (100) comprises:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,

- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body,
- to select a target region on the reference patient body and to identify a densely corresponding target regions of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,
- to read a saved six-dimensional pose and/or trajectory of the ultrasound probe (5) on the reference patient body from a computer-readable memory (30), and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body, and wherein at least one depth camera (6) is an RGB-D camera, a stereo camera, a time-of-flight camera or a LiDAR camera.

In a preferred embodiment, the ultrasound imaging system (100) comprises:

- a robotic arm (3) comprising an end effector (7),

- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body,
- to select a target region on the reference patient body and to identify a densely corresponding target regions of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,
- to read a saved six-dimensional pose and/or trajectory of the ultrasound probe (5) on the reference patient body from a computer-readable memory (30), and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body and wherein ultrasound probe pose determination unit (10) is configured to determine a six-dimensional pose and/or trajectory of the ultrasound probe (5) by the joint angle positions and arm positions of the robotic arm (3), from the depth image captured by the at least one depth camera (6).

In a preferred embodiment, the robotic arm (3) further comprises a force sensor system (8) to detect the contact pressure between the patient's skin and the ultrasound probe (5). The force sensor system (8) is preferably, an up to six dimensional force sensor system. Preferably, the force sensor system (8) is a six-dimensional force sensor system. Thus, the ultrasound imaging system (100) comprises:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body,
- to select a target region on the reference patient body and to identify a densely corresponding target regions of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,
- to read a saved six-dimensional pose and/or trajectory of the ultrasound probe (5) on the reference patient body from a computer-readable memory (30), and

- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body and

wherein the robotic arm (3) further comprises a force sensor system (8) and wherein the dense pose determination unit (20) is further configured to receive a contact pressure vector between the patient's skin and the ultrasound probe (5) recorded by the force sensor system (8), wherein the dense pose determination unit (20) is further configured to store the transformed six-dimensional position of the ultrasound probe (5) $p_{m,probe}$ together with the contact pressure vector in a computer-readable memory (30), and wherein the dense pose determination unit (20) is further configured to read the transformed six-dimensional position of the ultrasound probe (5) $p_{m,probe}$ together with the contact pressure vector in a computer-readable memory (30).

## Recording a position or trajectory of an ultrasound probe (5) relative to a patient's body

The inventive ultrasound imaging system (100) is particularly useful for transferring a 6D pose of the ultrasound probe from a reference patient to another patient and for transferring a 6D pose trajectory of the ultrasound probe from a reference patient to another patient.

Thus, another aspect of the present invention is directed to an ultrasound imaging system (100) comprising:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to read the reference patient body from the computer-readable memory (30),
- to receive one or more depth images of the patient's body captured with the at least one depth camera (6),
- optionally to fuse the one or more depth images to a single depth image,
- to estimate dense correspondences between the received depth image of the patient's body and a reference patient body,
- to select a target region on the reference patient body and to identify a densely corresponding target regions of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,
- to read a saved six-dimensional pose and/or trajectory of the ultrasound probe (5) on the reference patient body from a computer-readable memory (30), and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body and

wherein the system (100) is configured to store the 3D representation of at least the densely corresponding target region of the patient's body as the reference patient body and the determined six-dimensional pose and/or trajectory of the ultrasound probe (5) as the saved six-dimensional pose and/or trajectory of the ultrasound probe (5) on the reference patient body in the computer-readable memory (30).

In a preferred embodiment, the ultrasound imaging system (100) comprises:

- a robotic arm (3) comprising an end effector (7),
- an ultrasound probe (5) mounted on said end effector (7),

- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to read the reference patient body from the computer-readable memory (30),
- to receive one or more depth images of the patient's body captured with the at least one depth camera (6),
- optionally to fuse the one or more depth images to a single depth image,
- to estimate dense correspondences between the received depth image of the patient's body and a reference patient body by a surface detector,
- to select a target region on the reference patient body and to identify a densely corresponding target regions of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,
- to read a saved six-dimensional pose and/or trajectory of the ultrasound probe (5) on the reference patient body from a computer-readable memory (30), and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body, wherein the system (100) is configured to store the 3D representation of at least the densely corresponding target region of the patient's body as the reference patient body and the determined six-dimensional pose and/or trajectory of the ultrasound probe (5) as the saved six-dimensional pose and/or trajectory of the ultrasound probe (5) on the reference patient body in the computer-readable memory (30).

In a preferred embodiment, the ultrasound imaging system (100) comprises:

- a robotic arm (3) comprising an end effector (7) and a force sensor system (8),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to read the reference patient body from the computer-readable memory (30),
- to receive one or more depth images of the patient's body captured with the at least one depth camera (6),
- optionally to fuse the one or more depth images to a single depth image,
- to estimate dense correspondences between the received depth image of the patient's body and a reference patient body,
- to select a target region on the reference patient body and to identify a densely corresponding target regions of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,

- to read a saved six-dimensional pose and/or trajectory of the ultrasound probe (5) on the reference patient body from a computer-readable memory (30), and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body,

wherein the system (100) is configured to store the 3D representation of at least the densely corresponding target region of the patient's body as the reference patient body and the determined six-dimensional pose and/or trajectory of the ultrasound probe (5) as the saved six-dimensional pose and/or trajectory of the ultrasound probe (5) on the reference patient body in the computer-readable memory (30), and

wherein the system (100) is further configured to receive a contact pressure vector between the patient's skin and the ultrasound probe (5) recorded by the force sensor system (8), wherein the system (100) is further configured to store the transformed six-dimensional position of the ultrasound probe (5) $p_{m,probe}$ together with the contact pressure vector in a computer-readable memory (30), and wherein the system (100) is further configured to read the transformed six-dimensional position of the ultrasound probe (5) $p_{m,probe}$ together with the contact pressure vector from a computer-readable memory (30).

The inventive ultrasound imaging system (100) is also particularly useful for recording a 6D pose of the ultrasound probe relative to the patient and for recording a 6D pose trajectory of the ultrasound probe relative to the patient.

Thus, another aspect of the present invention is directed to an ultrasound imaging system (100) comprising:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body,
- to select a target region on the reference patient body and to identify a densely corresponding target regions of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,
- to read a saved six-dimensional pose and/or trajectory of the ultrasound probe (5) on the reference patient body from a computer-readable memory (30),
- to transform the determined six-dimensional pose and/or trajectory of the ultrasound probe (5) to the reference patient body using the function $f(p_{d,probe}) = p_{m,probe}$, wherein $p_{d,probe}$ is the determined six-dimensional pose and/or trajectory of the ultrasound probe (5) and $p_{m,probe}$ represents the corresponding six-dimensional pose and/or trajectory of the ultrasound probe (5) on the reference patient body, and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body, and

wherein the system is configured to store the transformed determined six-dimensional pose and/or trajectory of the ultrasound probe (5) in the computer-readable memory (30).

In a preferred embodiment, the ultrasound imaging system (100) comprises:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body by a surface detector,
- to select a target region on the reference patient body and to identify a densely corresponding target regions of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,
- to read a saved six-dimensional pose and/or trajectory of the ultrasound probe (5) on the reference patient body from a computer-readable memory (30),
- to transform the determined six-dimensional pose and/or trajectory of the ultrasound probe (5) to the reference patient body using the function $f(p_{d,probe}) = p_{m,probe}$, wherein $p_{d,probe}$ is the determined six-dimensional pose and/or trajectory of the ultrasound probe (5) and $p_{m,probe}$ represents the corresponding six-dimensional pose and/or trajectory of the ultrasound probe (5) on the reference patient body, and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body, and
wherein the system is configured to store the transformed determined six-dimensional pose and/or trajectory of the ultrasound probe (5) in the computer-readable memory (30).

In a preferred embodiment, the ultrasound imaging system (100) comprises:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5); wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body,
- to select a target region on the reference patient body and to identify a densely corresponding target regions of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by

regression of the selected target region and the densely corresponding target region, wherein $p_d$ is the densely corresponding target region in the depth image and $p_m$ is the selected target region on the reference patient body,

- to read a saved six-dimensional pose and/or trajectory of the ultrasound probe (5) on the reference patient body from a computer-readable memory (30),
- to transform the determined six-dimensional pose and/or trajectory of the ultrasound probe (5) to the reference patient body using the function $f(p_{d,probe}) = p_{m,probe}$, wherein $p_{d,probe}$ is the determined six-dimensional pose and/or trajectory of the ultrasound probe (5) and $p_{m,probe}$ represents the corresponding six-dimensional pose and/or trajectory of the ultrasound probe (5) on the reference patient body, and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body,
wherein the system is configured to store the transformed determined six-dimensional pose and/or trajectory of the ultrasound probe (5) in the computer-readable memory (30), and
wherein the system (100) is further configured to receive a contact pressure vector between the patient's skin and the ultrasound probe (5) recorded by the force sensor system (8), wherein the system (100) is further configured to store the transformed six-dimensional position of the ultrasound probe (5) $p_{m,probe}$ together with the contact pressure vector in a computer-readable memory (30), and wherein the system (100) is further configured to read the transformed six-dimensional position of the ultrasound probe (5) $p_{m,probe}$ together with the contact pressure vector from a computer-readable memory (30).

In a preferred embodiment, the ultrasound imaging system (100) comprises:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),

wherein the ultrasound probe pose determination unit (10) is configured to determine a six-dimensional pose and/or trajectory of the ultrasound probe (5);
wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body,
- to select a target region on the reference patient body and to identify a densely corresponding target regions of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,
- to read a saved six-dimensional pose and/or trajectory of the ultrasound probe (5) on the reference patient body from a computer-readable memory (30),
- to transform the determined six-dimensional pose and/or trajectory of the ultrasound probe (5) to the reference patient body using the function $f(p_{d,probe}) = p_{m,probe}$, wherein $p_{d,probe}$ is the determined six-dimensional pose and/or trajectory of the ultrasound probe (5) and $p_{m,probe}$ represents the corresponding six-dimensional pose and/or trajectory of the ultrasound probe (5) on the reference patient body, and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body,
wherein the system is configured to store the transformed determined six-dimensional pose and/or trajectory of the ultrasound probe (5) in the computer-readable memory (30), and wherein the reference patient body is a 3D human body surface model.

Another aspect of the present invention is directed to an ultrasound imaging system for recording a 6D ultrasound probe pose and/or trajectory relative to a patient's body and transferring the 6D ultrasound probe pose to a 3D human body surface model. Thus, the present invention is also directed to an ultrasound imaging system (100) comprising:

- an ultrasound probe (5),
- at least one depth camera (6) for capturing a depth image of a patient's body (1) and a surrounding, wherein the depth image comprises depth data and optionally color data,
- an ultrasound probe pose determination unit (10),
- a dense pose determination unit (20), and
- an ultrasound probe pose transformation unit (30),

wherein the ultrasound probe pose determination unit (10) is configured to determine a six-dimensional pose and/or trajectory of the ultrasound probe (5), wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a 3D human body surface model,
- to select a target region on the 3D human body surface model and to identify the densely corresponding target region of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the 3D human body surface model,
- to transform the determined six-dimensional pose and/or trajectory of the ultrasound probe (5) to the 3D human body surface model using the function $f(p_{d,probe}) = p_{m,probe}$, wherein $p_{d,probe}$ is the determined six-dimensional pose and/or trajectory of the ultrasound probe (5) and

$p_{m,probe}$ represents the corresponding six-dimensional pose and/or trajectory of the ultrasound probe (5) on the 3D human body surface model; and

wherein the system is configured to store the transformed six-dimensional pose of the ultrasound probe (5) $p_{m,probe}$ in a computer-readable memory (40).

Preferably, the 3D human body surface model used in the ultrasound imaging system (100) described herein is a 3D surface model of a generalised human, a 3D surface model of a man, a 3D surface model of a woman, or a 3D surface model of an individual body region.

In a preferred embodiment, the ultrasound imaging system (100) according to the present invention further comprises a display (50) configured to show a sonogram captured with the ultrasound probe (5).

In a preferred embodiment of the ultrasound imaging system (100) according to the present invention, the dense pose determination unit (20) is configured to select more than one target region on the 3D human body surface model and to identify the densely corresponding target regions of the patient's body in the one or more received depth image(s) and/or wherein the selected target region comprises a point of contact of the ultrasound probe (5).

In a preferred embodiment of the ultrasound imaging system (100) the coordinate transformation function f is a non-linear transformation or a linear transformation

$$f(p_d) = A \cdot p_d + t$$

wherein $A \in \mathbb{R}^{3x3}$ represents a transformation matrix and $t \in \mathbb{R}^3$ represents a translation part.

Another aspect of the present invention is directed to a **method for recording a position of an ultrasound probe (5) relative to a patient's body** (1) by using the inventive system (100).

Figure 1 shows a flow chart of the method. The method is preferably performed with a working ultrasound probe instead of an imitation, such that the physician is able to see the ultrasound image data when defining the desired poses in order to align the probe accordingly. The method allows trained persons, such as physicians, to "teach" desired 6D ultrasound probe poses on humans to the system in order to replay them again for subsequent patients. To this extent, a physician places an ultrasound probe on a patient in the position and orientation that he or she wants to record and store. The position and orientation of the ultrasound probe is recorded in global coordinates by the ultrasound probe position determination unit (10) as described herein. This can be achieved for example by attaching the probe directly to the robotic arm and knowing the joint angle positions of the end effector or by using markers on the probe.

Initially, a depth image of the patient's body is captured by the depth camera (6). The depth image may also contain color information. Using depth camera, 2D information can be transferred into 3D so that a 3D representation (e.g. 3D point cloud) is created from a recorded patient. This point cloud has the special feature that each point has a label corresponding to a body part. Dense correspondences between the 3D representation of the image and a 3D body surface model are estimated. A target region surrounding the ultrasound probe position is also selected from the depth image.

The acquisition of the initial 6D ultrasound probe pose and the dense body acquisition can be performed either sequentially or simultaneously. Sequential acquisition, however, requires that the patient does not move during the time between the body acquisition and the transducer pose acquisition (in either order). Simultaneous acquisition is still thus preferred, even when the target location is directly covered by the probe.

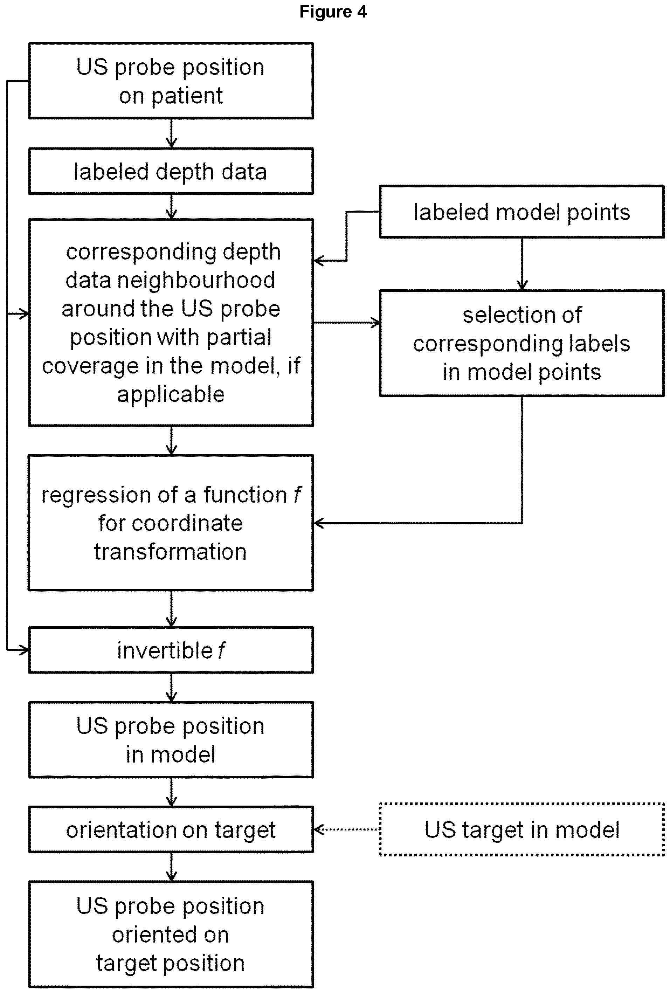

Subsequently, a coordinate transformation is determined for transferring the patient data to a 3D body surface model (see Figure 4), and stored in a computer-readable memory (30).

In a preferred embodiment the contact pressure vector is also recorded during the positioning. In another embodiment, ultrasound image data is also recorded.

Thus, the present invention is also directed to a method for recording a position of an ultrasound probe (5) relative to a patient's body (1) by using the ultrasound imaging system (100), the method comprising the following steps:

a) positioning and orienting the ultrasound probe (5) on the patient's body (1),
b) detecting a six-dimensional point of contact of the ultrasound probe (5) on the patient's body (1);
c) obtaining at least one depth image of the patient's body (1) from the at least one depth camera (6) and optionally fusing the at least one depth image to a single depth image, wherein the at least one depth image comprises depth data and optionally color data,
d) generating a 3D representation of the patient's body based on the obtained depth image;
e) storing the six-dimensional pose of the ultrasound probe (5) $p_{d,probe}$ and the 3D representation of the patient's body in a computer-readable memory (30), and
f) optionally repositioning and reorienting the ultrasound probe (5) and repeating steps b) to e), wherein in step e) the six-dimensional poses of the ultrasound probe (5) $p_{m,probe}$ are stored as a trajectory in the computer-readable memory (30).

In a preferred embodiment, the method for recording a position of an ultrasound probe (5) relative to a patient's body (1) by using the ultrasound imaging system (100), comprises the following steps:

a) positioning and orienting the ultrasound probe (5) on the patient's body (1),
b) detecting a six-dimensional point of contact of the ultrasound probe (5) on the patient's body (1);
c) obtaining at least one depth image of the patient's body (1) from the at least one depth camera (6) and optionally fusing the at least one depth image to a single depth image, wherein the at least one depth image comprises depth data and optionally color data,
d1) generating a 3D representation of the patient's body based on the obtained depth image,
d2) estimating dense correspondences between the 3D representation of the patient's body and a 3D representation of a reference patient body,
d3) selecting a target region on the 3D representation of a reference patient body,
d4) identifying a target region on the 3D representation of the patient's body densely corresponding to the selected target region in step d2),
d5) determining an invertible six-dimensional coordinate transformation function $f(p_d) = p_m$ by regression of the selected target region and the densely corresponding target region, wherein $p_d$ is the densely corresponding target region on the 3D representation of the patient's body and $p_m$ is the selected target region on the reference patient body,
d6) transforming the detected six-dimensional point of contact of the ultrasound probe (5) on the patient's body (1) to the 3D representation of the reference patient body using the function $f(p_{d,probe}) = p_{m,probe}$, wherein $p_{d,probe}$ is the six-dimensional position of the ultrasound probe (5) in the depth image and $p_{m,probe}$ represents the corresponding six-dimensional position of the ultrasound probe (5) in the 3D human body surface model;
e') storing the transformed six-dimensional pose of the ultrasound probe (5) $p_{m,probe}$ in a computer-readable memory (30), and
f) optionally repositioning and reorienting the ultrasound probe (5) and repeating steps b) to e), wherein in step e) the six-dimensional poses of the ultrasound probe (5) $p_{m,probe}$ are stored as a trajectory in the computer-readable memory (30),

wherein the 3D representation of a reference patient body is a 3D representation of a 3D human body surface model or a 3D representation generated from a depth image of another patient's body.

In a preferred embodiment, the method for recording a position of an ultrasound probe (5) relative to a patient's body (1) by using the ultrasound imaging system (100), comprises the following steps:

a) positioning and orienting the ultrasound probe (5) on the patient's body (1),

b) detecting a six-dimensional point of contact of the ultrasound probe (5) on the patient's body (1);

c) obtaining at least one depth image of the patient's body (1) from the at least one depth camera (6) and optionally fusing the at least one depth image to a single depth image, wherein the at least one depth image comprises depth data and optionally color data,

d1) generating a 3D point cloud of the patient's body based on the obtained depth image,

d2) estimating dense correspondences between the 3D point cloud of the patient's body and a 3D point cloud of a reference patient body,

d3) selecting a target region on the 3D point cloud of a reference patient body,

d4) identifying a target region on the 3D point cloud of the patient's body densely corresponding to the selected target region in step d2),

d5) determining an invertible six-dimensional coordinate transformation function $f(p_d) = p_m$ by regression of the selected target region and the densely corresponding target region, wherein $p_d$ is the densely corresponding target region on the 3D point cloud of the patient's body and $p_m$ is the selected target region on the reference patient body,

d6) transforming the detected six-dimensional point of contact of the ultrasound probe (5) on the patient's body (1) to the 3D point cloud of the reference patient body using the function $f(p_{d,probe}) = p_{m,probe}$, wherein $p_{d,probe}$ is the six-dimensional position of the ultrasound probe (5) in the depth image and $p_{m,probe}$ represents the corresponding six-dimensional position of the ultrasound probe (5) in the 3D human body surface model;

e') storing the transformed six-dimensional pose of the ultrasound probe (5) $p_{m,probe}$ in a computer-readable memory (30), and

f) optionally repositioning and reorienting the ultrasound probe (5) and repeating steps b) to e), wherein in step e) the six-dimensional poses of the ultrasound probe (5) $p_{m,probe}$ are stored as a trajectory in the computer-readable memory (30),

wherein the 3D point cloud of a reference patient body is a 3D point cloud of a 3D human body surface model or a 3D point cloud generated from a depth image of another patient's body.

In a preferred embodiment, the method for recording a position of an ultrasound probe (5) relative to a patient's body (1) stores a contact pressure vector for each ultrasound probe pose recorded by the (up to six-dimensional) force sensor system (8). Thus, the present invention is also directed to a method for recording a position of an ultrasound probe (5) relative to a patient's body (1) by using the ultrasound imaging system (100), the method comprising the following steps:

a) positioning and orienting the ultrasound probe (5) on the patient's body (1),

b1) detecting a six-dimensional point of contact of the ultrasound probe (5) on the patient's body (1);

b2) recording, by the force sensor system (8), a contact pressure vector between the patient's skin and the ultrasound probe (5);

c) obtaining at least one depth image of the patient's body (1) from the at least one depth camera (6) and optionally fusing the at least one depth image to a single depth image, wherein the at least one depth image comprises depth data and optionally color data,

d) generating a 3D representation of the patient's body based on the obtained depth image;

e) storing the six-dimensional pose of the ultrasound probe (5) $p_{d,probe}$, the 3D representation of the patient's body in a computer-readable memory (30) and the recorded contact pressure vector, and

f) optionally repositioning and reorienting the ultrasound probe (5) and repeating steps b) to e), wherein in step e) the six-dimensional poses of the ultrasound probe (5) $p_{m,probe}$ are stored as a trajectory in the computer-readable memory (30).

In a preferred embodiment, the method for recording a position of an ultrasound probe (5) relative to a patient's body (1) by using the ultrasound imaging system (100), comprises the following steps:

a) positioning and orienting the ultrasound probe (5) on the patient's body (1),

b1) detecting a six-dimensional point of contact of the ultrasound probe (5) on the patient's body (1);

b2) recording, by the force sensor system (8), a contact pressure vector between the patient's skin and the ultrasound probe (5);

c) obtaining at least one depth image of the patient's body (1) from the at least one depth camera (6) and optionally fusing the at least one depth image to a single depth image, wherein the at least one depth image comprises depth data and optionally color data,

d1) generating a 3D representation of the patient's body based on the obtained depth image,

d2) estimating dense correspondences between the 3D representation of the patient's body and a 3D representation of a reference patient body,

d3) selecting a target region on the 3D representation of a reference patient body,

d4) identifying a target region on the 3D representation of the patient's body densely corresponding to the selected target region in step d2),

d5) determining an invertible six-dimensional coordinate transformation function $f(p_d) = p_m$ by regression of the selected target region and the densely corresponding target region, wherein $p_d$ is the densely corresponding target region on the 3D representation of the patient's body and $p_m$ is the selected target region on the reference patient body,

d6) transforming the detected six-dimensional point of contact of the ultrasound probe (5) on the patient's body (1) to the 3D representation of the reference patient body using the function $f(p_{d,probe}) = p_{m,probe}$, wherein $p_{d,probe}$ is the six-dimensional position of the ultrasound probe (5) in the depth image and $p_{m,probe}$ represents the corresponding six-dimensional position of the ultrasound probe (5) in the 3D human body surface model;

e') storing the transformed six-dimensional pose of the ultrasound probe (5) $p_{m,probe}$ in a computer-readable memory (30) and the recorded contact pressure vector, and

f) optionally repositioning and reorienting the ultrasound probe (5) and repeating steps b) to e), wherein in step e) the six-dimensional poses of the ultrasound probe (5) $p_{m,probe}$ are stored as a trajectory in the computer-readable memory (30),

wherein the 3D representation of a reference patient body is a 3D representation of a 3D human body surface model or a 3D representation generated from a depth image of another patient's body.

In a preferred embodiment, the method for recording a position of an ultrasound probe (5) relative to a patient's body (1) by using the ultrasound imaging system (100), comprises the following steps:

a) positioning and orienting the ultrasound probe (5) on the patient's body (1),
b1) detecting a six-dimensional point of contact of the ultrasound probe (5) on the patient's body (1);
b2) recording, by the force sensor system (8), a contact pressure vector between the patient's skin and the ultrasound probe (5);
c) obtaining at least one depth image of the patient's body (1) from the at least one depth camera (6) and optionally fusing the at least one depth image to a single depth image, wherein the at least one depth image comprises depth data and optionally color data,
d1) generating a 3D point cloud of the patient's body based on the obtained depth image,
d2) estimating dense correspondences between the 3D point cloud of the patient's body and a 3D point cloud of a reference patient body,
d3) selecting a target region on the 3D point cloud of a reference patient body,
d4) identifying a target region on the 3D point cloud of the patient's body densely corresponding to the selected target region in step d2),
d5) determining an invertible six-dimensional coordinate transformation function $f(p_d) = p_m$ by regression of the selected target region and the densely corresponding target region, wherein $p_d$ is the densely corresponding target region on the 3D point cloud of the patient's body and $p_m$ is the selected target region on the reference patient body,

d6) transforming the detected six-dimensional point of contact of the ultrasound probe (5) on the patient's body (1) to the 3D point cloud of the reference patient body using the function $f(p_{d,probe}) = p_{m,probe}$, wherein $p_{d,probe}$ is the six-dimensional position of the ultrasound probe (5) in the depth image and $p_{m,probe}$ represents the corresponding six-dimensional position of the ultrasound probe (5) in the 3D human body surface model;

e') storing the transformed six-dimensional pose of the ultrasound probe (5) $p_{m,probe}$ in a computer-readable memory (30) and the recorded contact pressure vector, and

f) optionally repositioning and reorienting the ultrasound probe (5) and repeating steps b) to e), wherein in step e) the six-dimensional poses of the ultrasound probe (5) $p_{m,probe}$ are stored as a trajectory in the computer-readable memory (30),

wherein the 3D point cloud of a reference patient body is a 3D point cloud of a 3D human body surface model or a 3D point cloud generated from a depth image of another patient's body.

## Autonomous positioning and orienting of an ultrasound probe (5) on a patient's body

Another aspect of the present invention is directed to a method for autonomous positioning and orienting of an ultrasound probe (5) on a patient's body (1) by using the inventive system (100) described herein.

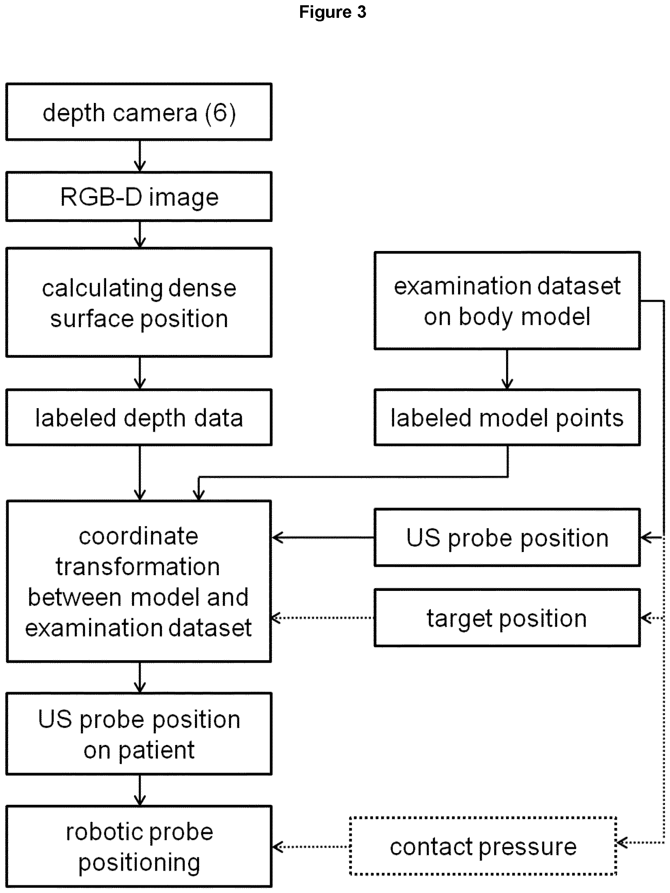

Figure 3 shows a flow chart of the method. Initially, a position or trajectory of the ultrasound probe (5) in the 3D human body surface model is provided, for example from a computer-readable memory (30). A depth image of the patient's body is captured by the depth camera (6). The depth image may also contain color information. Using a depth camera, 2D information can be transferred into 3D so that a 3D representation (e.g. point cloud) is created from a recorded patient. This point cloud has the special feature that each point has a label corresponding to a body part. Dense correspondences between the 3D representation of the image and a 3D body surface model are estimated. A target region surrounding the provided ultrasound probe position is also selected on the body model surface.

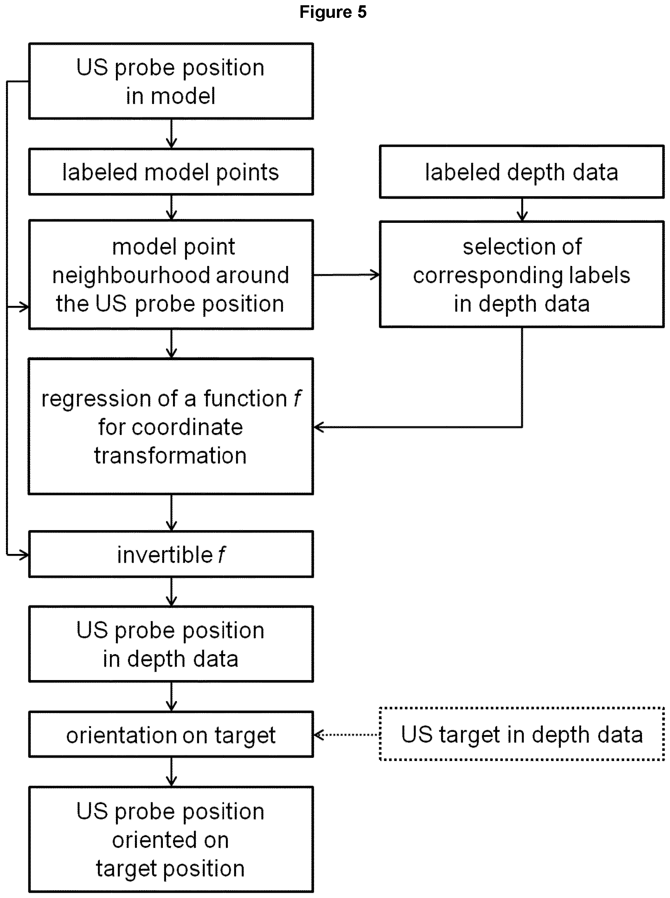

Subsequently, an invertible coordinate transformation is determined from the dense correspondences for transferring the patient data to a 3D body surface model. From the inverted coordinate transformation, the position or trajectory of the ultrasound probe (5) is transferred from the model coordinates to the depth image coordinates (see Figure 5). Finally, the robotic arm is controlled to position the ultrasound probe (5) at the transferred position and/or to execute the transferred trajectory of the ultrasound probe (5) on the patient.

Thus, the present invention is also directed to a method for autonomous positioning and orienting of an ultrasound probe (5) on a patient's body (1) by using the ultrasound imaging system (100) described herein, the method comprises the following steps:

a) reading a six-dimensional pose and/or trajectory of the ultrasound probe (5) on a 3D representation of a reference patient body from a computer-readable memory (30),

b) obtaining at least one depth image of the patient's body (1) from the at least one depth camera (6) and optionally fusing the at least one depth image to a single depth image,

c) generating a 3D representation of the patient's body based on the obtained depth image, wherein the at least one depth image comprises depth data and optionally color data;

d) estimating dense correspondences between the 3D representation of the patient's body and a 3D representation of a reference patient body,

e) selecting a target region on the 3D representation of the patient's body,

f) identifying a target region on the 3D representation of the reference patient body densely corresponding to the selected target region in step e),

g) determining an invertible six-dimensional coordinate transformation function $f(p_d) = p_m$ by regression of the selected target region and the densely corresponding target region, wherein $p_d$ is the selected target region on the 3D representation of the patient's body and $p_m$ is the densely corresponding target region on the reference patient body,

h) transforming the read six-dimensional pose and/or trajectory of the ultrasound probe (5) on a 3D representation of a reference patient body to the 3D representation generated ion step c) using the function $f^{-1}(p_{m,probe}) = p_{d,probe}$, wherein $f^{-1}$ is the inverted function of $f$, wherein $p_{d,probe}$ is the six-dimensional pose and/or trajectory of the ultrasound probe (5) on the 3D representation of the patient's body and $p_{m,probe}$ represents the read six-dimensional pose and/or trajectory of the ultrasound probe (5) on a 3D representation of a reference patient body;

i) positioning and orienting the ultrasound probe (5) at the transformed six-dimensional pose and/or trajectory on the patient's body (1) by the robotic arm (3).

In a preferred embodiment, the method for autonomous positioning and orienting of an ultrasound probe (5) on a patient's body (1) by using the ultrasound imaging system (100) described herein, comprises the following steps:

a1) reading a six-dimensional pose and/or trajectory of the ultrasound probe (5) on a 3D representation of a reference patient body from a computer-readable memory (30),

a2) reading a 3D representation of a reference patient body from a computer-readable memory (30),

b) obtaining at least one depth image of the patient's body (1) from the at least one depth camera (6) and optionally fusing the at least one depth image to a single depth image, wherein the at least one depth image comprises depth data and optionally color data,

c) generating a 3D representation of the patient's body based on the obtained depth image;

d) estimating dense correspondences between the 3D representation of the patient's body and a 3D representation of a reference patient body,

e) selecting a target region on the 3D representation of the patient's body,

f) identifying a target region on the 3D representation of the reference patient body densely corresponding to the selected target region in step e),

g) determining an invertible six-dimensional coordinate transformation function $f(p_d) = p_m$ by regression of the selected target region and the densely corresponding target region, wherein $p_d$ is the selected target region on the 3D representation of the patient's body and $p_m$ is the densely corresponding target region on the reference patient body,

h) transforming the read six-dimensional pose and/or trajectory of the ultrasound probe (5) on a 3D representation of a reference patient body to the 3D representation generated ion step c) using the function $f^{-1}(p_{m,probe}) = p_{d,probe}$, wherein $f^{-1}$ is the inverted function of $f$, wherein $p_{d,probe}$ is the six-dimensional pose and/or trajectory of the ultrasound probe (5) on the 3D representation of the patient's body and $p_{m,probe}$ represents the read six-dimensional pose and/or trajectory of the ultrasound probe (5) on a 3D representation of a reference patient body;

i) positioning and orienting the ultrasound probe (5) at the transformed six-dimensional pose and/or trajectory on the patient's body (1) by the robotic arm (3)

wherein the reference patient body is 3D representation generated from a depth image of another patient's body.

In a preferred embodiment, the method for autonomous positioning and orienting of an ultrasound probe (5) on a patient's body (1) by using the ultrasound imaging system (100) described herein, comprises the following steps:

a1) reading a six-dimensional pose and/or trajectory of the ultrasound probe (5) on a 3D point cloud of a reference patient body from a computer-readable memory (30),

a2) reading a 3D point cloud of a reference patient body from a computer-readable memory (30),

b) obtaining at least one depth image of the patient's body (1) from the at least one depth camera (6) and optionally fusing the at least one depth image to a single depth image, wherein the at least one depth image comprises depth data and optionally color data,

c) generating a 3D point cloud of the patient's body based on the obtained depth image;

d) estimating dense correspondences between the 3D point cloud of the patient's body and a 3D point cloud of a reference patient body,

e) selecting a target region on the 3D point cloud of the patient's body,

f) identifying a target region on the 3D point cloud of the reference patient body densely corresponding to the selected target region in step e),

g) determining an invertible six-dimensional coordinate transformation function $f(p_d) = p_m$ by regression of the selected target region and the densely corresponding target region, wherein $p_d$ is the selected target region on the 3D point cloud of the patient's body and $p_m$ is the densely corresponding target region on the reference patient body,

h) transforming the read six-dimensional pose and/or trajectory of the ultrasound probe (5) on a 3D point cloud of a reference patient body to the 3D point cloud generated ion step c) using the function $f^{-1}(p_{m,probe}) = p_{d,probe}$, wherein $f^1$ is the inverted function of $f$, wherein $p_{d,probe}$ is the six-dimensional pose and/or trajectory of the ultrasound probe (5) on the 3D point cloud of the patient's body and $p_{m,probe}$ represents the read six-dimensional pose and/or trajectory of the ultrasound probe (5) on a 3D point cloud of a reference patient body;

i) positioning and orienting the ultrasound probe (5) at the transformed six-dimensional pose and/or trajectory on the patient's body (1) by the robotic arm (3)

wherein the reference patient body is 3D point cloud generated from a depth image of another patient's body.

In a preferred embodiment, the robotic arm applies a previously recorded contact pressure on the ultrasound probe when positioning and orienting the ultrasound probe Thus, the present invention is also directed to a method for autonomous positioning and orienting of an ultrasound probe (5) on a patient's body (1) by using the ultrasound imaging system (100) described herein, the method comprises the following steps:

a') reading a six-dimensional pose and/or trajectory of the ultrasound probe (5) on a 3D representation of a reference patient body together with a contact pressure vector from a computer-readable memory (30),

b) obtaining at least one depth image of the patient's body (1) from the at least one depth camera (6) and optionally fusing the at least one depth image to a single depth image,

c) generating a 3D representation of the patient's body based on the obtained depth image, wherein the at least one depth image comprises depth data and optionally color data;

d) estimating dense correspondences between the 3D representation of the patient's body and a 3D representation of a reference patient body,

e) selecting a target region on the 3D representation of the patient's body,

f) identifying a target region on the 3D representation of the reference patient body densely corresponding to the selected target region in step e),

g) determining an invertible six-dimensional coordinate transformation function $f(p_d) = p_m$ by regression of the selected target region and the densely corresponding target region, wherein $p_d$ is the selected target region on the 3D representation of the patient's body and $p_m$ is the densely corresponding target region on the reference patient body,

h) transforming the read six-dimensional pose and/or trajectory of the ultrasound probe (5) on a 3D representation of a reference patient body to the 3D representation generated ion step c) using the function $f^{-1}(p_{m,probe}) = p_{d,probe}$, wherein $f^1$ is the inverted function of $f$, wherein $p_{d,probe}$ is the six-dimensional pose and/or trajectory of the ultrasound probe (5) on the 3D representation of the patient's body and $p_{m,probe}$ represents the read six-dimensional pose and/or trajectory of the ultrasound probe (5) on a 3D representation of a reference patient body;

i') positioning and orienting the ultrasound probe (5) at the transformed six-dimensional pose and/or trajectory on the patient's body (1) while applying the read contact pressure vector by the robotic arm (3).

In a preferred embodiment, the method for autonomous positioning and orienting of an ultrasound probe (5) on a patient's body (1) by using the ultrasound imaging system (100) described herein, comprises the following steps:

a1') reading a six-dimensional pose and/or trajectory of the ultrasound probe (5) on a 3D representation of a reference patient body together with a contact pressure vector from a computer-readable memory (30),

a2) reading a 3D representation of a reference patient body from a computer-readable memory (30),

b) obtaining at least one depth image of the patient's body (1) from the at least one depth camera (6) and optionally fusing the at least one depth image to a single depth image, wherein the at least one depth image comprises depth data and optionally color data,

c) generating a 3D representation of the patient's body based on the obtained depth image;

d) estimating dense correspondences between the 3D representation of the patient's body and a 3D representation of a reference patient body,

e) selecting a target region on the 3D representation of the patient's body,

f) identifying a target region on the 3D representation of the reference patient body densely corresponding to the selected target region in step e),

g) determining an invertible six-dimensional coordinate transformation function $f(p_d) = p_m$ by regression of the selected target region and the densely corresponding target region, wherein $p_d$ is the selected target region on the 3D representation of the patient's body and $p_m$ is the densely corresponding target region on the reference patient body,

h) transforming the read six-dimensional pose and/or trajectory of the ultrasound probe (5) on a 3D representation of a reference patient body to the 3D representation generated ion step c) using the function $f^{-1}(p_{m,probe}) = p_{d,probe}$, wherein $f^{-1}$ is the inverted function of $f$, wherein $p_{d,probe}$ is the six-dimensional pose and/or trajectory of the ultrasound probe (5) on the 3D representation of the patient's body and $p_{m,probe}$ represents the read six-dimensional pose and/or trajectory of the ultrasound probe (5) on a 3D representation of a reference patient body;

i') positioning and orienting the ultrasound probe (5) at the transformed six-dimensional pose and/or trajectory on the patient's body (1) while applying the read contact pressure vector by the robotic arm (3).

wherein the reference patient body is 3D representation generated from a depth image of another patient's body.

In a preferred embodiment, the method for autonomous positioning and orienting of an ultrasound probe (5) on a patient's body (1) by using the ultrasound imaging system (100) described herein, comprises the following steps:

a1') reading a six-dimensional pose and/or trajectory of the ultrasound probe (5) on a 3D representation of a reference patient body together with a contact pressure vector from a computer-readable memory (30),

a2) reading a 3D point cloud of a reference patient body from a computer-readable memory (30),

b) obtaining at least one depth image of the patient's body (1) from the at least one depth camera (6) and optionally fusing the at least one depth image to a single depth image, wherein the at least one depth image comprises depth data and optionally color data,

c) generating a 3D point cloud of the patient's body based on the obtained depth image;

d) estimating dense correspondences between the 3D point cloud of the patient's body and a 3D point cloud of a reference patient body,

e) selecting a target region on the 3D point cloud of the patient's body,

f) identifying a target region on the 3D point cloud of the reference patient body densely corresponding to the selected target region in step e),

g) determining an invertible six-dimensional coordinate transformation function $f(p_d) = p_m$ by regression of the selected target region and the densely corresponding target region, wherein $p_d$ is the selected target region on the 3D point cloud of the patient's body and $p_m$ is the densely corresponding target region on the reference patient body,

h) transforming the read six-dimensional pose and/or trajectory of the ultrasound probe (5) on a 3D point cloud of a reference patient body to the 3D point cloud generated ion step c) using the function $f^{-1}(p_{m,probe}) = p_{d,probe}$, wherein $f^{-1}$ is the inverted function of $f$, wherein $p_{d,probe}$ is the six-dimensional pose and/or trajectory of the ultrasound probe (5) on the 3D point cloud of the patient's body and $p_{m,probe}$ represents the read six-dimensional pose and/or trajectory of the ultrasound probe (5) on a 3D point cloud of a reference patient body;

i') positioning and orienting the ultrasound probe (5) at the transformed six-dimensional pose and/or trajectory on the patient's body (1) while applying the read contact pressure vector by the robotic arm (3).

wherein the reference patient body is 3D point cloud generated from a depth image of another patient's body.

**Description of the Figures**

**Figure 1:** shows a flow chart for recording an ultrasound scan relative to a patient's body with the system according to the invention. At least one depth camera is aligned with the patient to be scanned. The depth camera can be a stereo camera, a time-of-flight camera, a LiDAR or other depth-sensing hardware. The camera can optionally have a built-in grayscale or color camera, so that, if necessary, in addition to the depth information per pixel, color information is available with calibration. An algorithm for determining the dense surface position is applied to a camera image, either on the color data, a combination of color and depth data or only depth data. The algorithm calculates a position for each pixel that provides identical results when applied to different human bodies and allows a position comparison. The results are labeled depth data. The position of the ultrasound probe used for the examination is also recorded. The ultrasound probe can be located directly in the image of the depth camera or by an external system, e.g. a mechanical, an electromagnetic or an optical localization system. In the case of an external localization system, a device for time synchronization of the recordings, as well as of the transformation and the transformation determination between the coordinate system of the depth camera and the localization system is required, which is usually a method for hand-eye calibration. There may be an examination protocol that regulates a temporary fixation of the patient, so that, for example, a complete depth image of the patient is taken without partial coverage by the physician and ultrasound probe, and only then, with the patient's position assumed to be unchanged, the ultrasound probe is positioned on the patient. Recording patient's depth data and probe positioning can be performed separately. Optionally, data from a force sensor can be recorded synchronously to determine the current contact pressure vector between the ultrasound probe and the skin surface. Likewise, information about the target area to be scanned can optionally be stored synchronously. This can take the form of a depth estimate along the orientation of the ultrasound probe or precise positional information in the ultrasound volume and via a coordinate transformation or hand-eye calibration in ultrasound probe coordinates. The data sets recorded at a single point in time or over an examination interval, together with the transformation information between the different coordinate systems and time bases, are stored in an examination data set.

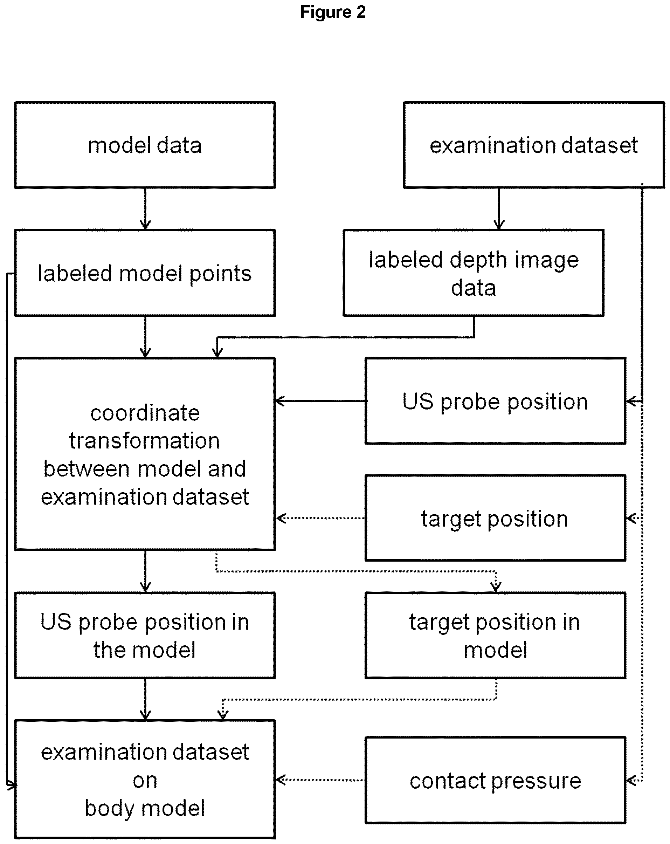

**Figure 2:** shows a flow chart for the transformation of a patient-specific ultrasound examination to a general body model using the system according to the invention. The body model is given in the form of a 3D model of a generalized human. Separate geometries for men and women can also be used. For special examinations, models of individual body regions may also be used. Labels in the form of position information exist for all model points, which allow an assignment between a labeled examination dataset and the corresponding model points at identical body positions. Typically, the model is of significantly higher resolution than the acquired depth data of the ultrasound examination, so that a unique assignment of a model point is possible for each label from the examination data set. Using the ultrasound probe position and, if necessary, a target position from the examination dataset, a local coordinate transformation is determined by regression, which converts labeled depth data and labeled model points around the transducer position into one another. This coordinate transformation is used to convert the probe position in the examination data set into a probe position on the model, consisting of transformation, point of contact and rotation, or probe orientation. If a target position for aligning the probe was given in the examination data set, this can also be transformed by the coordinate transformation to the model data and displayed relative to the model. The labeled model points for further coordinate transformations and the transducer pose in the model, as well as the target position in the model and the contact force from the original examination data set are combined into a new examination data set on the body model and output.

**Figure 3:** shows a flow chart for transforming a recorded ultrasound examination from a body model to a current patient by using the system according to the invention. During the recording of an ultrasound examination, at least one depth camera is aligned with the patient to be scanned. An algorithm is applied to the camera image to determine the dense surface position. It calculates position information for each image point, which allows a position comparison to other labeled model and examination data. The result is labeled depth data. An examination data set transferred to a body model contains the probe trajectory of an ultrasound examination and labeled model points of the body model. With the help of the probe position and, if available, a target position from the examination dataset, a local coordinate transformation is determined by regression, which converts labeled depth data and labeled model points around the probe position into one another. This coordinate transformation is used to convert the probe position in the examination data set into a probe position that is transformed to the current patient, consisting of a transformation, a point of contact and a rotation, or a probe orientation. The transformed probe position is transferred to the robotic arm. Optionally, a contact force from the examination data set, which the robot system has to exert on the patient at the current point is added to the transformed probe position.

**Figure 4:** shows a detailed flow chart for transforming an ultrasound probe position from the depth image data to a 3D body model by using the system according to the invention. From the recording of an ultrasound examination, both the six-dimensional probe position in rotation and translation and the labeled depth data of the patient are obtained preferably by a surface detector as a single image or time series. A labeled set of model points is also available, to which the probe position is to be transferred. To do this, a neighborhood around the probe position is first selected from the labeled depth data. If patient data and probe position are recorded separately one after the other, there are likely to be plenty of depth data in the area of around the transducer position (e.g. 5 centimeters), and these depth data points can be selected as the neighborhood. Particularly, in the case of partial coverage of the patient by the physician and the ultrasound probe, the selection of a suitable neighborhood represents a compromise between locality and visibility. The maximum distance between the transducer position and the depth data point is adjusted in such a way that a minimum number of depth data points in the neighborhood are distributed as well as possible around the probe position. The point set is transferred to the model by selecting the corresponding labels in the model points. The neighborhood relationships of the points can also be checked here. For example, the measure of surface distance can be used to ensure that all points are adjacent to each other and belong to the same body part. Outliers can be removed from both point sets. Then, a mathematical regression procedure is used to determine a transformation function f that makes a minimal error for the transformation of the point positions of the depth data into the positions of the corresponding model neighborhood. The function performs a complete, six-dimensional coordinate transformation and is invertible. Applied to the six-dimensional probe position on the patient, it can be used to calculate a six-dimensional probe position in the model. If a position of the ultrasound target is available in depth data and the orientation of the probe in the model is to be ensured for this position, the above procedure can additionally be carried out for the target position instead of the probe position, which results in a calculated ultrasound target in the model. The probe orientation is overwritten with the orientation towards the calculated ultrasound target and, together with the already determined probe translation, converted into a probe position in the model that is aligned with the target.

**Figure 5:** shows a detailed flow chart for transferring an ultrasound probe position from the model to a patient by using the system according to the invention. To this extent, the probe position in model coordinates is first used to determine a neighborhood of the labeled model points around its position. For example, by using the surface distance of the selected model points, it is ensured that the neighborhood contains only one contiguous body part. With the help of the label relationships, the corresponding point set in the depth data is determined for the created neighborhood, provided that the corresponding labels are present in the depth data. Subsequently, a mathematical regression procedure is used to determine a transformation function f that causes a minimal error for the transformation of the point positions of the model neighborhood into the positions of the corresponding depth data. The function performs a complete, six-dimensional coordinate transformation and is invertible. Applied to the six- dimensional probe position in the model, the function calculates a six-dimensional probe position in the depth data of the actual patient, i.e. on the patient himself, so that the robotic arm can move the probe to this position. If a position of the ultrasound target is available in model coordinates and the orientation of the probe is to be aligned with this position, the above procedure can be additionally carried out for the target position instead of the probe position with the result of a calculated ultrasound target in the current depth data. The probe orientation is overwritten with the alignment to the calculated ultrasound target.

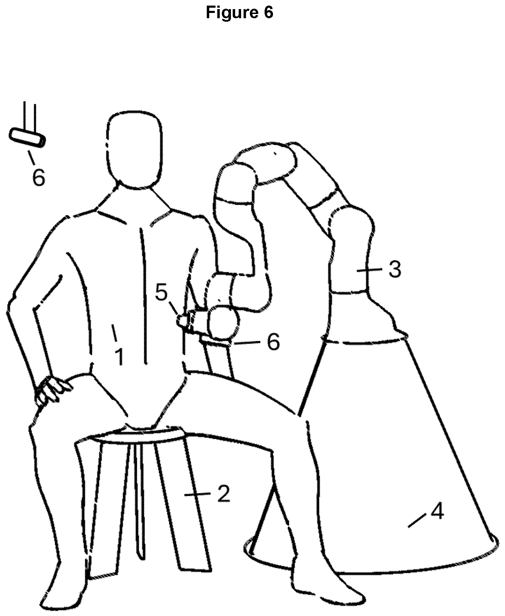

**Figure 6:** shows a human (1) placed on a pre-positioned seat (2), e.g. a chair, a couch or a special device for comfortable positioning. A robot kinematics (3), in this case a seven-axis industrial robot on a base (4) designed for handling people, can position an ultrasound probe / transducer (5) mounted on the end effector (7) on the human body. At least one depth camera (6) is mounted in the room or on the robot (eye-in-hand configuration) to record the environment. The examination is carried out in such a way that the robot is in a safe position. The depth camera is used to scan the surroundings for possible obstacles during the robot's movement and the position of the patient. If a depth camera is mounted on the robot, it may be useful to position the camera around the patient. For this purpose, a robot movement is planned and carried out taking into account static and detected obstacles. Based on the depth image data, a recorded examination sequence is transferred to the current patient. Again taking into account the obstacles, an approach, the examination trajectory itself and a descent movement of the robot kinematics into a safe position are planned and executed. Travelling movements are force-sensitive so that a collision with obstacles is detected and the robot kinematics can retract to a safe position or trigger an emergency stop if necessary. When following the trajectory, force control can be used to generate comparable, variable contact pressure forces over a limited distance in the approach direction of the transducer for the initial inspection.

The following examples are included to demonstrate preferred embodiments of the invention. It should be appreciated by those skilled in the art that the techniques disclosed in the examples, which follow represent techniques discovered by the inventor to function well in the practice of the invention, and thus can be considered to constitute preferred modes for its practice. However, those skilled in the art should, in light of the present disclosure, appreciate that many changes can be made in the specific embodiments, which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

**Reference Signs**

| | |
|---|---|
| 1 | human / patient |
| 2 | seat |
| 3 | robot kinematics / robotic arm |
| 4 | base |
| 5 | ultrasound probe / transducer |
| 6 | depth camera |
| 7 | end effector |
| 8 | force sensor system |
| 10 | ultrasound probe pose determination unit |
| 20 | dense pose determination unit |
| 30 | ultrasound probe pose transformation unit |
| 40 | computer-readable memory |
| 50 | display |
| 100 | ultrasound imaging system |

**Claims**

1. An ultrasound imaging system (100) comprising:

- a robotic arm (3) comprising an end effector (7),
- an ultrasound probe (5) mounted on said end effector (7),
- at least one depth camera (6) for capturing a depth image of a patient's body (1) and a surrounding, wherein the depth image comprises depth data and optionally color data,
- an ultrasound probe pose determination unit (10);
- a dense pose determination unit (20), and
- an ultrasound probe pose transformation unit (30),
wherein the ultrasound probe pose determination unit (10) is configured to determine a six-dimensional pose and/or trajectory of the ultrasound probe (5);
wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a reference patient body,
- to select a target region on the reference patient body and to identify a densely corresponding target region of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the reference patient body,
- to read a saved six-dimensional pose and/or trajectory of the ultrasound probe (5) on the reference patient body from a computer-readable memory (30), and
- to transform the read six-dimensional pose and/or trajectory of the ultrasound probe (5) on the reference patient body to the depth image using the function $f^{-1}(p_{m,probe}) = p_{d,probe}$, wherein $f^{-1}$ is the inverted function of $f$,

wherein the robotic arm (3) is configured to position the ultrasound probe (5) at the transformed six-dimensional pose and/or trajectory on the patient's body.

2. The ultrasound imaging system (100) according to claim 1, wherein the dense pose determination unit (20) is configured to estimate the dense correspondences between the received depth image of the patient's body and a reference patient body by a surface detector.

3. The ultrasound imaging system (100) according to claim 1 or 2, wherein the dense pose determination unit (20) is further configured to read the reference patient body from the computer-readable memory (30) and wherein the system is configured to store the 3D representation of at least the densely corresponding target region of the patient's body as the reference patient body and the determined six-dimensional pose and/or trajectory of the ultrasound probe (5) as the saved six-dimensional pose and/or trajectory of the ultrasound probe (5) on the reference patient body in the computer-readable memory (30).

4. The ultrasound imaging system (100) according to claim 1 or 2, wherein the ultrasound probe pose transformation unit (30) is further configured to transform the determined six-dimensional pose and/or trajectory of the ultrasound probe (5) to the reference patient body using the function $f(p_{d,probe}) = p_{m,probe}$, wherein $p_{d,probe}$ is the determined six-dimensional pose and/or trajectory of the ultrasound probe (5) and $p_{m,probe}$ represents the corresponding six-dimensional pose and/or trajectory of the ultrasound probe (5) on the reference patient body, wherein the system is configured to store the transformed determined six-dimensional pose and/or trajectory of the ultrasound probe (5) in the computer-readable memory (30).

5. The ultrasound imaging system (100) according to claim 3 or 4, wherein the robotic arm (3) further comprises a force sensor system (8) and wherein the system (100) is further configured to receive a contact pressure vector between the patient's skin and the ultrasound probe (5) recorded by the force sensor system (8), wherein the system (100) is further configured to store the transformed six-dimensional position of the ultrasound probe (5) $p_{m,probe}$ together with the contact pressure vector in a computer-readable memory (30), and wherein the system (100) is further configured to read the transformed six-dimensional position of the ultrasound probe (5) $p_{m,probe}$ together with the contact pressure vector from a computer-readable memory (30).

6. The ultrasound imaging system (100) according to any one of claims 1, 2, 4 and 5, wherein the reference patient body is a 3D human body surface model.

7. An ultrasound imaging system (100) comprising:

- an ultrasound probe (5),
- at least one depth camera (6) for capturing a depth image of a patient's body (1) and a surrounding, wherein the depth image comprises depth data and optionally color data,
- an ultrasound probe pose determination unit (10),
- a dense pose determination unit (20), and
- an ultrasound probe pose transformation unit (30),
wherein the ultrasound probe pose determination unit (10) is configured to determine a six-dimensional pose and/or trajectory of the ultrasound probe (5),
wherein the dense pose determination unit (20) is configured

- to receive one or more depth images of the patient's body captured with the at least one depth camera (6),
- optionally to fuse the one or more depth images to a single depth image,
- to estimate dense correspondences between the received depth image of the patient's body and a 3D human body surface model,

- to select a target region on the 3D human body surface model and to identify the densely corresponding target region of the patient's body in the received depth image, and
- to generate a 3D representation of at least the densely corresponding target region of the patient's body based on the received depth image,

wherein the ultrasound probe pose transformation unit (30) is configured

- to determine an invertible six-dimensional coordinate transformation function $f(p_d) = p_m$ by regression of the selected target region and the densely corresponding target region, wherein $p_d$ is the densely corresponding target region in the depth image and $p_m$ is the selected target region on the 3D human body surface model,
- to transform the determined six-dimensional pose and/or trajectory of the ultrasound probe (5) to the 3D human body surface model using the function $f(p_{d,probe}) = p_{m,probe}$, wherein $p_{d,probe}$ is the determined six-dimensional pose and/or trajectory of the ultrasound probe (5) and $p_{m,probe}$ represents the corresponding six-dimensional pose and/or trajectory of the ultrasound probe (5) on the 3D human body surface model; and

wherein the system is configured to store the transformed six-dimensional pose of the ultrasound probe (5) $p_{m,probe}$ in a computer-readable memory (40).

8. The ultrasound imaging system (100) according to claim 6 or 7, wherein the 3D human body surface model is a 3D surface model of a generalised human, a 3D surface model of a man, a 3D surface model of a woman, or a 3D surface model of an individual body region.

9. The ultrasound imaging system (100) according to any one of claims 1 to 8 further comprising a display (50) configured to show a sonogram captured with the ultrasound probe (5).

10. The ultrasound imaging system (100) according to any one of claims 1 to 9, wherein the dense pose determination unit (20) is configured to select more than one target region on the 3D human body surface model and to identify the densely corresponding target regions of the patient's body in the one or more received depth image(s) and/or wherein the selected target region comprises a point of contact of the ultrasound probe (5).

11. The ultrasound imaging system (100) according to any one of claims 1 to 10, wherein coordinate transformation function $f$ is a non-linear transformation or a linear transformation

$$f(p_d) = A \cdot p_d + t$$

wherein $A \in \mathbb{R}^{3x3}$ represents a transformation matrix and $t \in \mathbb{R}^3$ represents a translation part.

12. A method for recording a six-dimensional pose and/or trajectory of an ultrasound probe (5) relative to a patient's body (1) by using the ultrasound imaging system (100) according to any one of claims 3 to 11, the method comprising the following steps:

a) positioning and orienting the ultrasound probe (5) on the patient's body (1),
b) detecting a six-dimensional point of contact of the ultrasound probe (5) on the patient's body (1);
c) obtaining at least one depth image of the patient's body (1) from the at least one depth camera (6) and optionally fusing the at least one depth image to a single depth image wherein the at least one depth image comprises depth data and optionally color data,
d) generating a 3D representation of the patient's body based on the obtained depth image;
e) storing the six-dimensional pose of the ultrasound probe (5) $p_{d,probe}$ and the 3D representation of the patient's body in a computer-readable memory (30), and
f) optionally repositioning and reorienting the ultrasound probe (5) and repeating steps b) to e), wherein in step e) the six-dimensional poses of the ultrasound probe (5) $p_{m,probe}$ are stored as a trajectory in the computer-readable memory (30).

13. The method according to claim 12, wherein step d) comprises the following steps:

d1) generating a 3D representation of the patient's body based on the obtained depth image,
d2) estimating dense correspondences between the 3D representation of the patient's body and a 3D representation of a reference patient body,
d3) selecting a target region on the 3D representation of a reference patient body,
d4) identifying a target region on the 3D representation of the patient's body densely corresponding to the selected target region in step d2),

d5) determining an invertible six-dimensional coordinate transformation function $f(p_d) = p_m$ by regression of the selected target region and the densely corresponding target region, wherein $p_d$ is the densely corresponding target region on the 3D representation of the patient's body and $p_m$ is the selected target region on the reference patient body,

d6) transforming the detected six-dimensional point of contact of the ultrasound probe (5) on the patient's body (1) to the 3D representation of the reference patient body using the function $f(p_{d,probe}) = p_{m,probe}$, wherein $p_{d,probe}$ is the six-dimensional position of the ultrasound probe (5) in the depth image and $p_{m,probe}$ represents the corresponding six-dimensional position of the ultrasound probe (5) in the 3D human body surface model;

wherein the 3D representation of a reference patient body is a 3D representation of a 3D human body surface model or a 3D representation generated from a depth image of another patient's body, and

wherein step e) is replaced by step e'):

e') storing the transformed six-dimensional pose of the ultrasound probe (5) $p_{m,probe}$ in a computer-readable memory (30).

14. A method for autonomous positioning and orienting of an ultrasound probe (5) on a patient's body (1) by using the ultrasound imaging system (100) according to any one of claims 1 to 6 and 8 to 11, the method comprising the following steps:

a) reading a six-dimensional pose and/or trajectory of the ultrasound probe (5) on a 3D representation of a reference patient body from a computer-readable memory (30),

b) obtaining at least one depth image of the patient's body (1) from the at least one depth camera (6) and optionally fusing the at least one depth image to a single depth image, wherein the at least one depth image comprises depth data and optionally color data,

c) generating a 3D representation of the patient's body based on the obtained depth image;

d) estimating dense correspondences between the 3D representation of the patient's body and a 3D representation of a reference patient body,

e) selecting a target region on the 3D representation of the patient's body,

f) identifying a target region on the 3D representation of the reference patient body densely corresponding to the selected target region in step e),

g) determining an invertible six-dimensional coordinate transformation function $f(p_d) = p_m$ by regression of the selected target region and the densely corresponding target region, wherein $p_d$ is the selected target region on the 3D representation of the patient's body and $p_m$ is the densely corresponding target region on the reference patient body,

h) transforming the read six-dimensional pose and/or trajectory of the ultrasound probe (5) on a 3D representation of a reference patient body to the 3D representation generated ion step c) using the function $f^{-1}(p_{m,probe}) = p_{d,probe}$, wherein $f^{-1}$ is the inverted function of $f$, wherein $p_{d,probe}$ is the six-dimensional pose and/or trajectory of the ultrasound probe (5) on the 3D representation of the patient's body and $p_{m,probe}$ represents the read six-dimensional pose and/or trajectory of the ultrasound probe (5) on a 3D representation of a reference patient body;

i) positioning and orienting the ultrasound probe (5) at the transformed six-dimensional pose and/or trajectory on the patient's body (1) by the robotic arm (3).

15. The method according to claim 14, wherein step a) comprises

a1) reading a six-dimensional pose and/or trajectory of the ultrasound probe (5) on a 3D representation of a reference patient body from a computer-readable memory (30),

a2) reading a 3D representation of a reference patient body from a computer-readable memory (30),

wherein the reference patient body is 3D representation generated from a depth image of another patient's body.

**Figure 1**

**Figure 2**

**Figure 3**

**Figure 4**

**Figure 5**

**Figure 6**

## EUROPEAN SEARCH REPORT

**Application Number**

EP 24 21 5241

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MA XIHAN ET AL: "Autonomous Scanning Target Localization for Robotic Lung Ultrasound Imaging", 2021 IEEE/RSJ INTERNATIONAL CONFERENCE ON INTELLIGENT ROBOTS AND SYSTEMS (IROS), IEEE, 27 September 2021 (2021-09-27), pages 9467-9474, XP034051871, DOI: 10.1109/IROS51168.2021.9635902 [retrieved on 2021-12-03] * the whole document * | 1-15 | INV. A61B8/00 ADD. A61B34/20 |
| A | US 2022/405506 A1 (TAAMAZYAN VAGE [RU] ET AL) 22 December 2022 (2022-12-22) * figures 5, 6A * * paragraph [0069] - paragraph [0074] * * paragraph [0098] * | 1-15 | |

**TECHNICAL FIELDS SEARCHED (IPC)**

A61B

The present search report has been drawn up for all claims

| Place of search | Date of completion of the search | Examiner |
|---|---|---|
| Munich | 6 May 2025 | Willig, Hendrik |

EPO FORM 1503 03.82 (P04C01)

## ANNEX TO THE EUROPEAN SEARCH REPORT
## ON EUROPEAN PATENT APPLICATION NO.

EP 24 21 5241

This annex lists the patent family members relating to the patent documents cited in the above-mentioned European search report.
The members are as contained in the European Patent Office EDP file on
The European Patent Office is in no way liable for these particulars which are merely given for the purpose of information.

06-05-2025

| Patent document cited in search report | Publication date | Patent family member(s) | Publication date |
|---|---|---|---|
| US 2022405506 A1 | 22-12-2022 | US 2022405506 A1 | 22-12-2022 |
| | | WO 2022271831 A1 | 29-12-2022 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7889906 B

**Non-patent literature cited in the description**

- **YE, RUIZHONG et al.** Feasibility of a 5G-based robot-assisted remote ultrasound system for cardiopulmonary assessment of patients with coronavirus disease 2019. *Chest*, 2021, vol. 159 (1), 270-281
- **SHIDA** ; **YUUKI et al.** Automated image acquisition of parasternal long-axis view with robotic echocardiography. *IEEE Robotics and Automation Letters*, 2023
- Concept Towards Segmenting Arm Areas for Robot-Based Dermatological In Vivo Measurements.. **SZYMANSKI** ; **MATEUSZ et al.** 2nd German-West African Conference on Sustainable, Renewable Energy Systems (SusRES2021).. TIB Open Publishing, 2021
- **CAO** ; **ZHE et al.** Realtime multi-person 2d pose estimation using part affinity fields.. *Proceedings of the IEEE conference on computer vision and pattern recognition.*, 2017
- **LOPER, MATTHEW et al.** SMPL: A skinned multi-person linear model. *Seminal Graphics Papers: Pushing the Boundaries*, 2023, vol. 2, 851-866
- Keep it SMPL: Automatic estimation of 3D human pose and shape from a single image. **BOGO, FEDERICA et al.** Computer Vision-ECCV 2016: 14th European Conference, Amsterdam, The Netherlands, October 11-14, 2016, Proceedings. Springer International Publishing, 11 October 2016
- **GÜLER** ; **RIZA ALP** ; **NATALIA NEVEROVA** ; **IASONAS KOKKINOS**. Densepose: Dense human pose estimation in the wild.. *Proceedings of the IEEE conference on computer vision and pattern recognition.*, 2018
- **WANG** ; **JINBAO et al.** Deep 3D human pose estimation: A review. *Computer Vision and Image Understanding*, 2021, vol. 210, 103225